(19) Europäisches Patentamt
European Patent Office
Office européen des brevets

(11) EP 3 504 180 B1

(12) FASCICULE DE BREVET EUROPEEN

(45) Date de publication et mention de la délivrance du brevet:
**23.03.2022 Bulletin 2022/12**

(21) Numéro de dépôt: **17768869.4**

(22) Date de dépôt: **24.08.2017**

(51) Classification Internationale des Brevets (IPC):
***C07C 67/08*** *(2006.01)* ***C08G 63/66*** *(2006.01)*
***C08G 63/668*** *(2006.01)* ***C08G 18/42*** *(2006.01)*

(52) Classification Coopérative des Brevets (CPC):
(C-Sets disponibles)
**C08G 18/42; C07C 69/40; C07C 69/44; C08G 18/4244; C08G 63/668;** C08G 2110/0008; C08G 2110/0016; C08G 2110/0025 (Cont.)

(86) Numéro de dépôt international:
**PCT/IB2017/055110**

(87) Numéro de publication internationale:
**WO 2018/037373 (01.03.2018 Gazette 2018/09)**

# (54) MOUSSE RIGIDE COMPRENANT UN POLYOL POLYESTER

HARTSCHAUM MIT EINEM POLYESTERPOLYOL

RIGID FOAM COMPRISING A POLYESTER POLYOL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.08.2016 FR 1601253**

(43) Date de publication de la demande:
**03.07.2019 Bulletin 2019/27**

(73) Titulaires:
- **Tereos Starch & Sweeteners Belgium**
  **9300 Aalst (BE)**
- **Centre National de la Recherche Scientifique**
  **75794 Paris Cedex 16 (FR)**
- **Société Soprema SAS**
  **67025 Strasbourg (FR)**
- **Université de Strasbourg**
  **67081 Strasbourg Cedex (FR)**

(72) Inventeurs:
- **BINDSCHEDLER, Pierre Etienne**
  **67025 Strasbourg Cedex (FR)**
- **SARBU, Alexandru**
  **67025 Strasbourg Cedex (FR)**
- **LAURICHESSE, Stephanie**
  **67025 Strasbourg Cedex (FR)**
- **PERRIN, Remi**
  **67025 Strasbourg Cedex (FR)**
- **FURTWENGLER, Pierre**
  **75794 Paris Cedex 16 (FR)**
- **AVÉROUS, Luc**
  **75794 Paris Cedex 16 (FR)**
- **REDL, Andreas**
  **77230 Moussy-le-Vieux (FR)**

(74) Mandataire: **Icosa**
**83 avenue Denfert-Rochereau**
**75014 Paris (FR)**

(56) Documents cités:
**US-A- 2 863 855** **US-A- 4 001 180**
**US-A- 4 404 295**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

(52) Classification Coopérative des Brevets (CPC): (Cont.)

C-Sets
**C07C 67/08, C07C 69/34**

## Description

### Domaine technique

La présente invention concerne une mousse rigide polyuréthane comprenant un polyol polyester pouvant être d'origine biosourcée.

### Arrière-plan technique

Les polyuréthanes (PU) sont des polymères polyvalents et sont utilisés dans différentes applications telles que l'automobile, le mobilier, la construction, les chaussures, l'isolation acoustique et thermique avec une production mondiale de 18 Mt en 2016, en plaçant le PU au 6$^{ième}$ rang parmi les polymères basé sur les résultats annuels Production mondiale.

De nos jours, l'industrie du PU dépend fortement de composants pétrosourcés tel que les polyols polyéthers obtenus par réaction d'alcoxylation. Les isocyanates sont historiquement obtenus à partir de la chimie du phosgène ou du diphosgène. Selon diverses législations, en particulier le protocole de Kyoto en Europe, il est maintenant obligatoire de réduire les émissions de gaz à effet de serre de la production à l'utilisation finale d'un produit. Un exemple très illustratif de cela est l'attention croissante portée à l'isolation des bâtiments, en particulier la « bio-isolation » de locaux individuels et collectifs. L'un des meilleurs matériaux pour l'isolation des bâtiments est la mousse de polyuréthane rigide (PUR), basée sur la polyaddition des polyols et de polyisocyanurates à haute fonctionnalité portant 2 à 3 groupes isocyanate pour obtenir des matériaux à cellules fermées. La conductivité thermique des mousses PUR varie entre 20 mW / (mK) et 30 mW / (mK) contre 30 mW / (mK) et 40 mW / (mK) pour le polystyrène expansé (EPS) ou 40 mW / (mK) et 50 mW / (MK) pour le polystyrène extrudé (XPS).

Les mousses PUR rivalisent maintenant avec des mousses rigides polyisocyanurate-polyuréthane (PUIR) qui présentent des performances supérieures aux mousses PUR classiques. Les mousses PUIR sont basées sur la trimérisation des diisocyanates à haute température en cycle isocyanurate aussi appelé anneau triisocyanurique (Schéma 1) en présence d'un catalyseur spécifique. La formulation de mousse PUIR est légèrement différente des mousses PUR. Un excès de fonction isocyanate est requis pour obtenir des anneaux d'isocyanurate trifonctionnels.

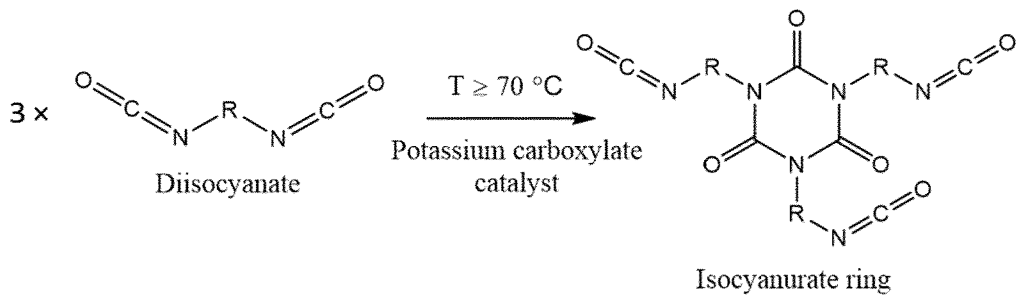


Schéma 1 : Trimérisation des diisocyanate en présence de catalyseur à base de carboxylate de potassium

Ainsi, un polyol de fonctionnalité inférieure peut être utilisé. Le réseau de mousses PUIR repose sur une double chimie. Le polyol réagit avec l'isocyanate pour former du polyuréthane. Ensuite, les polyisocyanates excédentaires trimérisent en cycle isocyanurate à l'origine de la forte densité de réticulation de la mousse finale. La forte densité de réticulation des mousses PUIR constitue leur principal inconvénient, car elle induit de la friabilité au matériel.

La friabilité de mousses PUIR est largement compensée par leurs propriétés supérieures vis-à-vis des mousses PUR, en particulier par leur résistance thermique plus élevée. Il a été établi que la gamme de stabilité thermique de la fonction uréthane dépend de leur environnement chimique et évolue entre 120°C et 250°C. La plage de stabilité thermique de la fonction isocyanurate dépend également de la fonction chimique environnante, mais est estimée entre 365°C et 500°C. La meilleure stabilité thermique des fonctions isocyanurate présentes dans les mousses PUIR est à l'origine de leur meilleure résistance au feu par rapport aux mousses PUR. La résistance thermique des mousses PUIR par rapport aux mousses PUR les rend vraiment attrayantes dans le secteur de l'isolation des bâtiments. Les secteurs du bâtiment et de la construction font face à de nouvelles normes de résistance thermique et au feu de plus en plus drastiques vis-à-vis des matériaux utilisés. En dépit de ces propriétés supérieures, peu de recherches ont été effectuées sur le système PUIR en fonction de la substitution du polyol pétrosourcé par un polyol à base de sorbitol issu de la biomasse ou une formulation contenant 100% de polyol renouvelable. Récemment, seule l'huile de colza, le glycérol brut, l'huile de ricin, les microalgues et les polyols à base de tanins ont été utilisés dans la mousse PUR-PUIR.

Les propriétés des mousses PUIR sont principalement liées à leur morphologie et à leur structure interne, ce qui a un effet significatif sur la conductivité thermique et les propriétés mécaniques. Il est bien établi que les propriétés thermiques des matériaux en mousse dépendent principalement de la teneur en cellules fermées et du gaz qu'ils contiennent (H. Fleurent and S. Thijs, J. Cell. Plast., 1995, 31, 580-599). Il est également bien admis que les propriétés mécaniques des matériaux expansés dépendent étroitement de leur densité. J. Mills (N. J. Mills, J. Cell. Plast., 2011, 47, 173-197) a étudié les mousses de polyéthylène et de polystyrène à cellules fermées et a montré que l'air inclus dans les cellules contribuait de manière significative à la résistance aux compressions des mousses à faible densité. Néanmoins, les propriétés mécaniques des mousses PUIR ne sont pas souvent très étudiées. J. Andersons *et al.* (J. Andersons et al., Mater. Des., 2016, 92, 836-845) ont travaillé sur des mousses polyisocyanurate partiellement biosourcées, à faible densité et à cellules fermées. Ils ont étudié l'anisotropie de la résistance à la compression des mousses entre la direction longitudinale et transversale à la montée de la mousse. Ils ont montré que le rapport entre les modules de Young et la force dans la direction longitudinale et la direction transversale est respectivement d'environ 3 et 1,4.

Le document US 4 404 295 A décrit des compositions de polyester polyols utilisés pour préparer des mousses de polyuréthane (colonne 1, l.10 15). L'exemple 3 décrit la préparation d'un polyester polyol à partir d'acide adipique, d'anhydride phtalique, de diéthylène glycol et de glycérol. Dans la préparation de ce polyol polyester en une seule étape, un sucre, deux diols (identiques) et deux acides sont utilisées.

La présente invention vise à développer une nouvelle mousse PUIR préparée à partir de produits biosourcés et plus particulièrement d'un polyester polyol biosourcé susceptible de remplacer les polyols pétrosourcés utilisés pour les mousses du marché dans leur application traditionnelle. L'objectif de la présente invention est de proposer une mousse biosourcée présentant des propriétés mécaniques et physiques comparable aux mousses pétrosourcés, par exemple en termes de taille de cellules, de dégradation thermique, de cinétique, de moussage, de dureté, de compressibilité, de densité ou de conductivité thermique.

## Description détaillée de l'invention

L'invention concerne une mousse rigide ou une composition permettant l'obtention d'une mousse rigide comprenant un polyol polyester obtenu par une première polycondensation (a) d'un sucre alcool Z en C3 à C8 et de deux diacides Y et Y' identiques ou différents en C4 à C36 et d'une seconde polycondensation (b) du produit obtenu en (a) avec deux diols X et X' identiques ou différents en C2 à C12.

L'invention concerne en outre une mousse rigide ou une composition permettant l'obtention d'une mousse rigide comprenant un polyol polyester de formule générale **Rx-Ry-Z-Ry'-Rx'** dans laquelle

- **Z** est un sucre alcool en C3 à C8, préférentiellement en C4 à C7, typiquement en C5, C6,
- **Ry** et **Ry'** sont des diesters de formule $-OOC-C_n-COO-$ avec n compris entre 2 et 34, préférentiellement entre 3 et 22, typiquement entre 4 et 10,
- **Rx** et **Rx'** sont des monoalcools, identiques ou différents en C2 à C12, préférentiellement en C3 à C8, typiquement en C4.

Typiquement, le terme « mousse » tel qu'utilisé par exemple dans les expressions « mousse de polyuréthane » ou « mousse polyisocyanurate », on entend un composé à structure alvéolaire tridimensionnelle de type expansé. Ladite mousse peut être rigide ou souple, à cellules ouvertes ou fermées.

On entend par « mousse rigide » une mousse présentant une bonne résistance à la compression et dont la structure interne est irréversiblement endommagée lors d'une déformation en compression comprise entre 5 et 50%. Généralement de telles mousses présentent des températures de transition vitreuse (Tg) supérieur à 100°C souvent proche de 200°C. Les mousses rigides sont généralement des mousses à haut taux de cellules fermées (généralement supérieur à 90%).

On parle de polyuréthane rigide (PUR), ou de polyisocyanurate rigide (PUIR) pour des mousses rigides de polyuréthane ou de polyisocyanurate.

Par « mousse à cellules fermées » on entend une mousse dont la structure alvéolaire comporte des parois entre chaque alvéole constituant un ensemble de cellules jointes et distinctes permettant l'emprisonnement d'un gaz d'expansion. Une mousse est qualifiée de mousse à cellules fermées lorsqu'elle présente un maximum de 10% de cellules ouvertes. Typiquement les mousses à cellules fermées sont majoritairement des mousses rigides.

Par « mousse à cellules ouvertes » on entend une mousse dont la structure alvéolaire est constituée d'une matrice alvéolaire continue à parois ouverte entre les cellules ne permettant pas l'emprisonnement d'un gaz d'expansion. Une telle mousse permet la création de chemins de percolation au sein de sa matrice alvéolaire. Typiquement, les mousses à cellules ouvertes sont majoritairement des mousses souples ou semi-rigides.

Le terme « polyol polyester » fait référence à des molécules comprenant des groupements hydroxyles (diols ou sucres alcools) liés entre eux par des liaisons esters. Ainsi, dans le polyol polyester selon l'invention, les molécules

X, Y, Z, Y'et X' sont liées entre elles par des liaisons esters. Typiquement, les diols X et X' et le sucre alcool Z sont liés aux deux diacides Y et Y' par des liaisons esters formées chacune entre une fonction acide de Y ou de Y' et une fonction hydroxyle primaire de Z, X ou X'. Avantageusement, le polyol polyester est à pH neutre, typiquement lorsqu'il est obtenu par deux polycondensations successives suivies d'une étape de neutralisation (par exemple à la potasse ou à la soude).

Le polyol polyester selon l'invention présente avantageusement la formule chimique générale $C_aH_bO_c$ avec $22 \leq a \leq 42$, $38 \leq b \leq 78$, $14 \leq c \leq 22$.

Typiquement, le polyol polyester selon l'invention présente une masse moléculaire comprise entre 350 g/mol et 2000 g/mol, préférentiellement entre 420 g/mol et 1800 g/mol, et plus préférentiellement entre 450 et 1700 g/mol. Selon l'invention, la masse molaire du polyol polyester peut être déterminée par différentes méthodes telles que la chromatographie d'exclusion stérique.

Avantageusement, le polyol polyester présente un indice hydroxyle de 300 à 900 mg KOH/g. L'indice hydroxyle (IOH) peut être calculé avec la formule suivante :

IOH = fonctionnalité du polyol polyester x 56109.37 / Masse molaire du polyol polyester

L'indice hydroxyle correspond au nombre de mg de KOH nécessaire pour déprotoner l'ensemble des groupements hydroxyle présents dans un gramme de polyol. L'indice d'hydroxyle peut être déterminé par dosage inverse utilisant de la potasse, par exemple selon la norme ASTM 4274-99 dans laquelle la titration colorimétrique est remplacée par une titration pH-métrique.

Par « sucre alcool » ou « polyol » on entend une forme hydrogénée de monosaccharide dont le groupe carbonyle (aldéhyde ou cétone) a été réduit en un hydroxyle primaire ou secondaire. Typiquement, le sucre alcool est choisi parmi le glycérol, le sorbitol, l'érythritol, le xylitol, l'arabitol, le ribitol, le dulcitol, le mannitol et le volémitol.

Par « diacide » on entend une chaîne carbonée comprenant deux groupements acide. Selon l'invention, le polyol polyester comprend deux molécules Y et Y' de diacide. Ces molécules peuvent être identiques ou différentes en C4 à C36, préférentiellement C4 à C24. Typiquement, les deux molécules de diacide sont indépendamment choisies parmi l'acide butanedioïque (Acide succinique), l'acide pentanedioïque (Acide glutarique), l'acide hexanedioïque (Acide adipique), l'acide heptanedioïque (Acide pimélique), l'acide octanedioïque (Acide subérique), l'acide nonanedioïque (Acide azélaïque), l'acide décanedioïque (Acide sébacique), l'acide undécanedioïque, l'acide dodécanedioïque, l'acide tridécanedioïque (Acide brassylique), l'acide tétradécanedioïque, l'acide pentadécanedioïque, l'acide hexadécanedioïque, dimères d'acides gras ayant jusqu'à 36 carbones (C36) ou leur mélange. Typiquement, Y et Y' sont des diacides en C5 à C16 ou C6 à C12. Avantageusement, les molécules de diacide préférées sont indépendamment choisies parmi l'acide adipique et l'acide succinique.

Par «diol » on entend une chaîne carbonée comprenant deux groupements alcool. Selon l'invention, le polyol polyester comprend deux molécules X et X' de diols identiques ou différentes. Typiquement, les molécules de diol sont indépendamment choisies parmi le 1,2-éthanediol, le 1,3-propanediol, le 1,4-butanediol, le 1,6-hexanediol, le 1,8-octanediol, le 1,10-décanediol, le 1,12-dodécanediol et leur mélange.

Avantageusement, le polyol polyester selon l'invention est choisi parmi le bis(1,2-éthanediol)-sorbitol diadipate, le bis(1,3-propanediol)-sorbitol diadipate, le bis(1,4-butanediol)-sorbitol diadipate, le bis(1,4-butanediol)-sorbitol diadipate modifié avec du glycérol, le bis(1,6-hexanediol)-sorbitol diadipate, le bis(1,8-octanediol)-sorbitol diadipate, le bis(1,10-décanediol)-sorbitol diadipate, le bis(1,12-dodécanediol)-sorbitol diadipate, le bis(1,4-butanediol)-sorbitol disuccinate et le sorbitol-diadipate-sorbitol. Préférentiellement, ledit polyol polyester est choisi parmi le bis(1,8-octanediol)-sorbitol diadipate, le bis(1,10-décanediol)-sorbitol diadipate et le bis(1,4-butanediol)-sorbitol diadipate.

L'invention concerne également une mousse rigide ou une composition permettant l'obtention d'une mousse rigide comprenant un polyol polyester obtenu par un procédé comprenant les étapes suivantes :

a) une étape de polycondensation à une température comprise entre 110 et 200°C, préférentiellement 120 à 180°C, plus préférentiellement 130 et 170°C, typiquement 150°C, avantageusement durant 5 à 10 heures:

i. d'un sucre alcool Z en C3 à C8, préférentiellement en C4 à C7, avantageusement en C5-C6, typiquement choisi parmi le glycérol, le sorbitol, l'érythritol, le xylitol, l'arabitol, le ribitol, le dulcitol, le mannitol et le volémitol,
ii. de deux diacides Y et Y' identiques ou différents en C4 à C36, préférentiellement en C5 à C24,
iii. de deux diols X et X' identiques ou différents en C2 à C12, préférentiellement en C3 à C8, typiquement en C4, avantageusement indépendamment choisis parmi le 1,2-éthanediol, le 1,3-propanediol, le 1,4-butanediol, le 1,6-hexanediol, le 1,8-octanediol, le 1,10-décanediol, le 1,12-dodécanediol, le 1,4-butanediol et leur mélange,

b) optionnellement, une étape de neutralisation des fonctions acides libres de sorte à ramener le polyol polyester

à un pH neutre (pH=7), par exemple, par une base, typiquement une base forte telle que de la potasse, ou par une base faible telle que le carbonate de sodium, le bicarbonate de sodium, le carbonate de potassium ou un mono bi ou trialcool en C4 à C8, tel que de l'hexanol ; préférentiellement l'étape de neutralisation est effectuée par ajout de carbonate de potassium ou de la potasse.

Avantageusement, lors de l'étape de polycondensation, les diols X et X' et le sucre alcool Z sont à un ratio molaire (X+X')/Z compris entre 1 et 3, préférentiellement entre 1,5 et 2,5, encore plus préférentiellement compris entre 1,8 et 2,2.

Typiquement lors de l'étape de polycondensation, les diacides Y et Y' et le sucre alcool Z sont à un ratio molaire (Y+Y')/Z compris entre 1 et 3, préférentiellement entre 1,5 et 2,5, encore plus préférentiellement compris entre 1,8 et 2,2.

Selon un mode de réalisation, lors de l'étape de polycondensation, les diols X et X' et les diacides Y et Y' sont à un ratio molaire (X+X')/(Y+Y') compris entre 0,5 et 2, préférentiellement entre 0,7 et 1,5, encore plus préférentiellement compris entre 0,8 et 1,2.

Avantageusement, l'étape de polycondensation comprend une première polycondensation (a) du sucre alcool Z et des diacides Y et Y' et une seconde polycondensation (b) du produit obtenu en (a) avec les diols X et X'. Cette polycondensation en deux étapes permet l'obtention du polyol polyester avec cette structure symétrique. Typiquement, les diacides Y et Y' sont identiques et/ou les diols X et X' sont identiques.

Selon un mode de réalisation, le sucre alcool Z est mélangé avec la ou les molécules de diacide Y et Y' puis incubé durant plus d'une heure, plus préférentiellement entre 2 et 5 heures, encore plus préférentiellement entre 2,5 et 4h, typiquement durant 3 heures. La ou les molécules de diol X et X' sont ajoutées dans un second temps au mélange puis incubées durant plus de 4 heures, préférentiellement entre 5 et 10 heures, typiquement entre 5,5 et 7h. De manière préférentielle, l'étape de polycondensation est effectuée sous vide.

Avantageusement, lors de l'étape de polycondensation, les molécules de diacide Y et Y' réagissent avec les alcools primaires des molécules de sucre alcool Z puis des diols X et X'. Les molécules d'eau issues de la réaction sont récupérées en vue de leur élimination.

L'invention concerne en outre une mousse rigide ou une composition permettant l'obtention d'une mousse rigide comprenant un polymère comprenant le polyol polyester selon l'invention, typiquement ledit polymère est un polyuréthane et/ou un polyisocyanurate.

Avantageusement, le polymère selon l'invention présente une masse molaire supérieure à $1{,}7.10^6$ g/mol. Typiquement, le polymère est un polymère réticulé.

Par « polyuréthane », on entend un polymère comprenant des fonctions uréthanes autrement dit, un polymère d'uréthane. Ces polymères résultent essentiellement de la réaction de polyols notamment du polyol polyester de l'invention et de polyisocyanates. Ces polymères sont généralement obtenus à partir de formulations présentant un index de 100 à 150, préférentiellement de 105 à 130, correspondant à un rapport NCO/OH compris entre 1 et 1,5, préférentiellement entre 1,05 et 1,3.

Par « polyisocyanurate », on entend les polymères résultant de la réaction de polyols notamment du polyol polyester de l'invention et de polyisocyanates, qui contiennent, en plus des liaisons d'uréthane, d'autres types de groupes fonctionnels, en particulier des anneaux triisocyanuriques formés par trimérisation des polyisocyanates. Ces polymères, normalement appelés aussi les polyuréthanes modifiés ou polyisocyanurate-polyuréthane, sont généralement obtenus à partir de formulations présentant un index 150 à 700, préférentiellement entre 200 et 500, encore plus préférentiellement entre 250-400, soit un rapport NCO/OH compris entre 1,5 et 7, préférentiellement entre 2,0 et 5,0 préférentiellement entre 2,5 et 4,0.

Selon l'invention, ledit polymère est un mélange de polyuréthane et de polyisocyanurate. Un tel mélange est observé par exemple lorsque ledit polymère comprend des fonctions uréthanes et des polyisocyanates trimérisés en anneaux triisocyanuriques. Typiquement, ledit polymère est un mélange de polyuréthane et de polyisocyanurate et présente un index supérieur à 100 ou inférieur ou égal à 400, correspondant à un rapport NCO/OH supérieur à 1 ou inférieur ou égal à 4.

Par ratio NCO/OH on entend, au sens de la présente invention, le rapport entre le nombre de fonctions NCO du polyisocyanate et le nombre de fonctions OH du polyol polyester, des co-polyols et de tout autre composant comportant des groupements OH (eau, solvants) présent dans une formulation. Le ratio NCO/OH est calculé avec la formule suivante :

$$\text{Ratio NCO / OH} = M_{exp}Pi \times ME\ Pi / M_{exp}\ SAI \times ME\ SAI$$

où :

- $M_{exp}Pi$ est la masse du polyisocyanate ;
- $M_{exp}SAI$ est la masse du sucre alcool ;

- ME SAI est la masse équivalente du sucre alcool et correspond au ratio entre la masse molaire du sucre alcool et la fonctionnalité du sucre alcool ;
- MEPi est la masse équivalente du polyisocyanate et correspond au ratio entre la masse molaire du polyisocyanate et la fonctionnalité du polyisocyanate.

Par « liaison urée» on entend, au sens de la présente invention une liaison urée disubstituée soit le produit de la réaction entre une amine primaire et une fonction isocyanate d'un polyisocyanate. Les amines primaires peuvent être introduites dans la composition ou sont le produit de la réaction entre une molécule d'eau et une fonction isocyanate d'un polyisocyanate.

Typiquement, ladite mousse rigide ou composition permettant l'obtention de ladite mousse rigide comprenant ledit polyol polyester selon l'invention ou ledit polymère selon l'invention notamment le prépolymère, comprend en outre un catalyseur de réaction, un polyisocyanate ayant une fonctionnalité au moins égale à 2, un stabilisant, un agent gonflant, optionnellement, un co-polyol et des additifs.

Par « co-polyol », on entend un composé portant deux fonctions hydroxyles (type diol) ou plus (polyol) ajouté à la composition comprenant le polyol polyester pour en ajuster les propriétés telle que la fonctionnalité ou la viscosité, pour créer des nœuds de réticulation ou être allongeur de chaîne. La mousse selon l'invention comprend au moins un co-polyol en C2 à C8, préférentiellement en C2 à C7, avantageusement en C2 à C6. Le au moins un co-polyol peut être avantageusement choisis parmi l'éthylène glycol, le glycérol, le 1,4-butanediol, le butane-1,3-diol, 1,3-propanediol, le propane-1,2-diol, 1,5-pentanediol, 1,6-hexanediol, 1,2-propylene glycol, 3-oxapentane-1,5-diol, 2-[2-(2-hydroxyéthoxy)éthoxy]éthanol, le benzène-1,2,4-triol, le benzène 1,2,3-triol, le benzène 1,3,5-triol, le sorbitol, l'érythritol, le xylitol, l'arabitol, le ribitol, le dulcitol, le mannitol et le volémitol, préférentiellement le au moins un co-polyol est choisi parmi le glycérol, l'éthylène glycol, le 1,4-butanediol, 1,3-propanediol, 1,5-pentanediol, 1,2-propylene glycol, 3-oxapentane-1,5-diol, et le sorbitol, le au moins un co-polyol préféré est choisi parmi le glycérol, l'éthylène glycol, le 1,4-butanediol et le sorbitol.

Typiquement, le(s) co-polyol(s) est ajouté dans un ratio polyol polyester/co-polyol(s) de 70/30 à 99/1, préférentiellement 75/25 à 95/5, encore plus préférentiellement 80/20 et 92/8, typiquement 82/8 et 90/10, par exemple 85/15.

Selon l'invention, la composition comprend deux co-polyols typiquement un co-polyol en C2 et un co-polyol en C3 ou un co-polyol en C2 et un co-polyol en C4 ou un co-polyol en C2 et un co-polyol en C5 ou un co-polyol en C2 et un co-polyol en C6 ou un co-polyol en C3 et un co-polyol en C5 ou un co-polyol en C3 et un co-polyol en C4 ou un co-polyol en C3 et un co-polyol en C6 ou un co-polyol en C5 et un co-polyol en C6, ou deux co-polyols en C3 ou deux co-polyols en C5 ou deux co-polyols en C6.

Avantageusement, la composition comprend au moins un co-polyol en C2, typiquement, deux co-polyols, par exemple un co-polyol en C2 et un co-polyol en C3 ou C4 ou C5 ou C6, typiquement, l'éthylène glycol et le glycérol, l'éthylène glycol et l'érythritol, l'éthylène glycol et le xylitol, l'éthylène glycol et l'arabitol, l'éthylène glycol et le ribitol, l'éthylène glycol et le dulcitol, l'éthylène glycol et le mannitol, l'éthylène glycol et le 1,4-butanediol, l'éthylène glycol et le 1,3-propanediol, le 1,3-propanediol et le 1,4-butanediol, ou l'éthylène glycol et le volémitol.

Avantageusement, la composition comprend deux co-polyols, typiquement dans un ratio C2/C6 ou C2/C5 ou C2/C6 ou C2/C3 ou C3/C6 ou C3/C5 ou C5/C6 compris entre 95/05 à 50/50, préférentiellement 90/10 à 55/45, préférentiellement 87/13 à 60/40, plus préférentiellement 85/15 à 62/38, encore plus préférentiellement 80/20 à 65/35.

Par « polyisocyanate », on entend tout composé chimique comprenant au moins deux fonctions chimiques isocyanates (NCO) distinctes, autrement dit, ayant «une fonctionnalité au moins égale à 2 ». Lorsque le polyisocyanate à une fonctionnalité de 2, on parle de di-isocyanate. Par fonctionnalité, on entend, au sens de la présente invention, le nombre total de fonctions isocyanate réactives par molécule d'isocyanate. La fonctionnalité d'un produit est évaluée par la titration de la fonction NCO par une méthode de dosage en retour de la dibultylamine excédentaire par l'acide chlorhydrique. Typiquement, ledit polyisocyanate a une fonctionnalité entre 2 et 5, préférentiellement entre 2,5 et 3,5, encore plus préférentiellement entre 2,7 et 3,3. Avantageusement, ledit polyisocyanate est choisi parmi des polyisocyanates aromatiques, aliphatiques, cycloaliphatiques et leurs mélanges. On peut citer par exemple le 2,4-toluène diisocyanate, le 2,6-toluène diisocyanate, les mélanges de 2,4- et 2,6-toluène diisocyanate, le m-phénylène diisocyanate, le p-phénylène diisocyanate, le cis / trans de cyclohexane diisocyanate hexaméthylène diisocyanate, le m- et le p-tétraméthylxylylène-diisocyanate, m-xylylène, p-xylylène diisocyanate, le naphtalène-m, m-diisocyanate, le 1,3,5-hexaméthyl mésitylène triisocyanate, 1-méthoxyphényl-2,4-diisocyanate, 4,4'-diphényl-mehtane diisocyanate, 4,4'-diisocyanabiphénylène 3,3'-diméthoxy-4,4'-diphényl diisocyanate, 3,3'-dimethyl-4,4'-diphényl diisocyanate, 4,4",4"-triphénylméthane triisocyanate, le toluène-2,4,6m-triisooyanate, 4,4'-diméthyl diphényl méthane-2,2', 5,5'-tétraisocyanate, et les isocyanates aliphatiques, tels que le 4,4'-diphénylméthane diisocyanate hydrogéné, toluène diisocyanate (TDI) hydrogénée et hydrogenatem méta- et paraxylène diisocyanate de tétraméthylxylylène diisooyanate (TMXDI®isooyanate, produit de American Cyanamid co, Wayne, NJ, USA.), 3: 1 méta-tétraméthylxylylène diisocyanate / triméthylolpropane (Cythane 3160® isocyanate, de la société American Cyanamid Co.), les molécules plurifonctionnelles tel que le poly-diisocyanate de diphénylméthylène (pMDI) et leurs analogues. Typiquement le polyisocyanate est choisi parmi le toluène diisocyanate

(TDI), 4,4'-diphénylméthane diisocyanate (ou 4,4'-diisocyanate de diphénylméthylène ou 4,4'-MDI), le polyméthylène polyphénylène polyisocyanate (MDI polymérique, pMDI) et leur mélange.

Par « catalyseur de réaction », on entend un composé qui introduit en faible quantité accélère la cinétique de la formation de la liaison uréthane (-NH-CO-O-) par réaction entre le polyol polyester de l'invention et un polyisocyanate ou active la réaction entre un polyisocyanate et l'eau ou active la trimérisation des isocyanates. Typiquement les catalyseurs de réaction sont choisis parmi des amines tertiaires (tel que le diméthylcyclohexane), des dérivés d'étain (tel que le dibutyldilaurate d'étain), sels d'ammoniums (tel que le méthanaminium N,N,N-triméthyl de 2,2-dimethylpropanoate) des carboxylates de métaux alcalins (tel que le 2-éthylhexanoate de potassium ou l'octoate de potassium) les éthers d'amine (tel que le bis(2-dimethylaminoéthyle) éther), et les triazines (tel que le 1,3,5-Tris(3-(dimethylamino)propyl))hexahydro-1,3,5-triazine).

Avantageusement, une composition destinée à l'obtention d'une mousse comprend ledit polyol polyester selon l'invention ou ledit polymère selon l'invention notamment le pré-polymère, au moins un catalyseur de réaction, au moins un polyisocyanate ayant une fonctionnalité au moins égale à 2, au moins un agent gonflant, un stabilisant et optionnellement un ignifugeant, un co-polyol.

Avantageusement, lorsque la composition est une mousse ou une composition permettant l'obtention d'une mousse, le polyol polyester préféré est un polyol polyester à pH neutre et/ou comprend un sorbitol au titre de sucre-alcool Z. Typiquement, le polyol polyester préféré est le bis(1,2-éthanediol)-sorbitol-diadipate, le bis(1,6-hexanediol)-sorbitol-diadipate ou le bis(1,4-butanediol)-sorbitol-diadipate, plus préférentiellement, le bis(1,4-butanediol)-sorbitol-diadipate, ou le bis(1,6-hexanediol)-sorbitol-diadipate.

Selon l'invention, une mousse comprend après polymérisation, typiquement, un polymère selon l'invention notamment un polymère réticulé, au moins un catalyseur de réaction, au moins un agent gonflant, un stabilisant et optionnellement, au moins un co-polyol.

Par « agent gonflant » on entend, un composé induisant par une action chimique et/ou physique une expansion d'une composition au cours d'une étape de moussage. Typiquement, l'agent gonflant chimique est choisi parmi l'eau, l'acide formique, l'anhydride phtalique et l'acide acétique L'agent gonflant physique est choisi parmi le pentane et les isomères du pentane, les hydrocarbures, les hydrofluorocarbures, les hydrochlorofluorooléfines, les hydrofluoro-oléfines (HFOs), les éthers et leurs mélanges. On peut citer le méthylal au titre d'exemple d'un agent gonflant de type éther. Selon l'invention, un mélange d'agent gonflant chimique et physique préféré est par exemple un mélange eau/isomère du pentane ou acide formique/isomère du pentane ou eau/hydrofluoro-oléfines ou isomère de pentane/méthylal/eau ou encore eau/méthylal.

Par « stabilisant » on entend, un agent permettant la formation d'une émulsion entre le polyol et l'agent gonflant, la nucléation des sites d'expansion de l'agent gonflant, ainsi que la stabilité physique de la matrice polymère lors de l'avancement des réactions. Typiquement, les stabilisants sont choisis parmi l'un quelconque des copolymères silicone glycol (par exemple Dabco DC198 ou DC193 commercialisé par Air Products), copolymère non hydrolysable silicone glycol (par exemple DC5000from Air Products), copolymère de polyalkylène siloxane (par exemple Niax L-6164 de Momentive), copolymère méthylsiloxane polyoxyalkylène (par exemple Niax L-5348 de Momentive), copolymère polyétherpolysiloxane (par exemple Tegostab B8870 ou Tegostab B1048 de Evonik), copolymère de polydiméthylsiloxane polyéther (par exemple Tegostab B8526 de Evonik), polyéthersiloxane (par exemple Tegostab B8951 de Evonik), un copolymère polyéther-polysiloxane modifié (par exemple Tegostab B8871 de Evonik), un copolymère de polysiloxane polyoxyalkylene à block (par exemple de Tegostab BF 2370 de Evonik) et leurs dérivés ou leurs mélanges.

Par « additifs », on entend des agents tels que des anti-oxydants (agents de neutralisation des extrémités de chaînes à l'origine de la dépolymérisation ou chaînes co-monomères capables d'arrêter la propagation de dépolymérisation), des agents démoulant (talc, solution paraffine, silicone), des anti-hydrolyses, des biocides, des agents anti-UV (l'oxyde de titane, les triazines, les benzotriazoles) et/ou des ignifugeants (antimoine, composés phosphorées, borées, azotées).

Par « agent ignifugeant », on entend un composé ayant la propriété de réduire ou d'empêcher la combustion ou l'échauffement des matériaux qu'elle imprègne ou recouvre, on parle de retardant de flamme ou de feu. On peut citer par exemple seul ou en mélange, le graphite, les silicates, le bore, les dérivés halogénés ou phosphorés tels que le Tris (1-chloro-2-propyl) phosphate (TCPP), le triéthylène de phosphate (TEP), les esters phosphate de triaryle, le polyphosphate d'ammonium, le phosphore rouge, le trishalogénaryle, et leur mélange.

Un exemple de composition selon l'invention permettant l'obtention d'une mousse polyuréthane rigide à cellules fermées est typiquement formulée avec un index compris entre 101 et 200, préférentiellement entre 102 et 170, encore plus préférentiellement entre 105 et 150 par exemple de 115, soit un rapport NCO/OH compris entre 1,01 et 2, préférentiellement entre 1,02 et 1,7, encore plus préférentiellement entre 1,05 et 1,5 par exemple de 1.2.

Typiquement, une telle composition comprend :

- au moins 1 à 100 parts, préférentiellement de 40 à 100 parts, encore plus préférentiellement entre 80 à 100 parts, d'un polyol polyester selon l'invention,

- 0 à 70 parts, préférentiellement de 1 à 50 parts, encore plus préférentiellement entre 2 et 30 parts, d'au moins un co-polyol,
- 150 à 500 parts, préférentiellement de 160 à 425 parts, encore plus préférentiellement entre 180 et 375 parts, d'au moins un polyisocyanate,
- 0,5 à 5 parts d'au moins un catalyseur, typiquement d'un catalyseur aminé tel que la diméthylcyclohexylamine,
- 0,5 à 15 parts d'au moins un agent gonflant, typiquement 0,5 à 12 parts, préférentiellement 0,6 à 10 parts, encore plus préférentiellement 0,7 à 9 parts, d'un agent gonflant chimique tel que l'eau et/ou 0 à 60 parts, préférentiellement 0,5 à 30 parts, encore plus préférentiellement 1 à 25 parts, d'un agent gonflant physique tel que les dérivés d'isopentane,
- 0 à 5 parts d'un stabilisant tel qu'un copolymère polyéther-polysiloxane et
- 0 à 20 parts d'un agent ignifugeant.

Une mousse de polyuréthane rigide à cellules fermées comprend par exemple 100 parts d'un polyol polyester, 270 parts d'un polyisocyanate, 2 parts d'un catalyseur aminé tel que la diméthylcyclohexylamine, 6 parts d'un agent gonflant tel que l'eau, 2,5 parts d'un stabilisant tel qu'un copolymère polyéther-polysiloxane et 10 parts d'un agent ignifugeant.

Un exemple d'une composition permettant l'obtention d'une mousse rigide polyisocyanurate à cellules fermées est typiquement formulée avec un index minimum de 200 soit un ratio NCO /OH supérieur à 2.0, préférentiellement un index compris entre 250 et 450, encore plus préférentiellement compris entre 300 et 400, soit un ratio NCO/OH préférentiellement compris entre 2,5 et 4,5, encore plus préférentiellement compris entre 3,0 et 4,0.

Une composition permettant l'obtention d'une mousse rigide polyisocyanurate à cellules fermées comprend

- 60 à 100 parts, préférentiellement de 70 à 100 parts, encore plus préférentiellement entre 80 et 100 parts, du polyol polyester selon l'invention,
- 0 à 50 parts, préférentiellement de 1 à 40 parts encore plus préférentiellement entre 5 et 20 parts d'un co-polyol,
- 100 à 700 parts, préférentiellement de 120 à 650 parts, encore plus préférentiellement entre 150 et 575 parts, d'au moins un polyisocyanate,
- 0,1 à 13 parts, préférentiellement de 0.5 à 12 parts, encore plus préférentiellement entre 1 et 11 parts, d'au moins un catalyseur, préférentiellement deux catalyseurs, typiquement un catalyseur aminé et un carboxylate de potassium (par exemple dans un ratio catalyseur aminé / carboxylate de potassium de 0,2 à 2),
- 0 à 80 parts, préférentiellement de 5 à 70 parts, encore plus préférentiellement entre 10 et 60 parts, d'au moins un agent gonflant tel qu'un isomère du pentane,
- 0 à 8 parts, préférentiellement de 1 à 7 parts, encore plus préférentiellement entre 1,5 et 6 parts, d'un stabilisant,
- 0 à 30 parts, préférentiellement de 5 à 25 parts, encore plus préférentiellement entre 10 et 20 parts, d'un agent ignifugeant.

Typiquement, une composition permettant l'obtention d'une mousse rigide polyisocyanurate à cellules fermées comprend par exemple, 85 parts du polyol polyester selon l'invention ; 15 parts d'un co-polyol tel que l'éthylène glycol ; 550 parts d'un polyisocyanate tel que le poly-diisocyanate de diphénylméthylène ; 1,6 parts d'un catalyseur aminé tel que la bis(2-dimethylaminoethyl)éther ; 7 parts d'un carboxylate de potassium tel que par exemple le 2-ethylhexanoate de potassium ; 0,8 parts d'une triazine tel que la 1,3,5-tri(3-[dimethylamino]propyl)-hexahydro-s-triazine ; 45 parts d'un agent gonflant tel qu'un isomère du pentane ; 2,5 parts d'un stabilisant et 15 parts d'un agent ignifugeant.

L'invention concerne également un panneau ou un bloc de mousse rigide comprenant la mousse rigide de l'invention, typiquement pour l'isolation thermique ou phonique notamment des bâtiments ou l'isolation cryogénique des réfrigérateurs, cuve de bateaux gazier, ou pour le comblement ou l'aide à la flottaison tel que dans les outils d'aide à la flottaison (ceinture ou gilet..) ou de sports nautiques.

On entend par « panneau » ayant approximativement une forme parallélépipède rectangle présentant des surfaces relativement lisses et les dimensions suivantes de 0,3 à 50 $m^2$ de surface pour une épaisseur de 10 à 1000 mm, préférentiellement de 0,5 à 20 $m^2$ de surface pour une épaisseur de 15 à 500 mm ; encore plus préférentiellement de 0,8 à 15 $m^2$ de surface pour une épaisseur 17 à 400 mm, typiquement, de 1 à 7 $m^2$ de surface pour une épaisseur 20 à 250 mm. Des exemples de dimensions sont, typiquement, une surface de 600 $\times$ 600 mm ou 1200 $\times$ 600 mm pour une épaisseur 20 à 250 mm.

On entend par bloc une structure de toute forme géométrique, cubique parallélépipédique, en étoile ou cylindrique, avec ou sans évidement(s), d'un volume compris entre, 1$cm^3$ à 100 $m^3$, préférentiellement 10$cm^3$ à 70 $m^3$, encore plus préférentiellement 100 $cm^3$ à 50 $m^3$, typiquement 0,5 à 35 $m^3$, typiquement, de 1 à 30 $m^3$.

L'invention concerne également une méthode d'obtention de panneau ou un bloc de mousse rigide selon l'invention.

L'invention concerne une méthode d'isolation thermique, phonique ou cryogénique notamment de bâtiments,

de conduites de transport de fluides ou une méthode de comblement (de fissures ou d'espace libre), d'étanchéification (de structures, de fissures..), de scellage ou d'amélioration de la flottaison (typiquement d'outils d'aide à la flottaison ou de sports nautiques) par le dépôt ou l'introduction de blocs ou de panneaux de mousse selon l'invention ou par la projection d'une mousse rigide ou d'une composition permettant l'obtention d'une mousse rigide selon l'invention.

L'invention concerne également, un procédé d'obtention d'une mousse rigide typiquement de polyuréthane ou de polyisocyanurate comprenant :

- une étape d'obtention d'un polyol polyester selon l'invention ou d'un polymère selon l'invention notamment d'un prépolymère selon l'invention,
- une étape d'addition d'au moins un polyisocyanate, d'au moins un agent gonflant, d'un stabilisant et d'au moins un catalyseur de réaction, et
- une étape de polymérisation.

Bien qu'ayant des significations distinctes, les termes « comprenant », « contenant », « comportant » et « consistant en» ont été utilisés de manière interchangeable dans la description de l'invention, et peuvent être remplacés l'un par l'autre. L'invention sera mieux comprise à la lecture des figures et exemples suivants donnés uniquement à titre d'exemple.

## FIGURES

**Figure 1** : Profil de moussage PUIR pétrosourcé : a. température, b. vitesse d'expansion, c. hauteur maximale normalisée de la mousse

**Figure 2** : Évolution de la température du milieu réactionnel pendant le moussage de la référence et des deux mousses biosourcées B1-KO et B2-PC

**Figure 3** : Aspect des mousses PUIR : a) 100/0, b) B1-K0, c) B2-PC

**Figure 4** : Clichés MEB de la mousse PUIR 100/0 : a) dans la direction transversale à l'expansion de la mousse, b) dans la direction longitudinale à l'expansion de la mousse **Figure 5** : Spectre FTIR des mousses PUIR 100/0, 35/65, B1-KO et B2-PC

## Exemples

### I. Matériel et méthode

### a. Produits chimiques

Le polyester polyol pétrosourcé est un polyester aromatique à base d'anhydride phtalique modifié de STEPANP (STEPANPOL® PS-2412), appelé polyol pétrosourcé. Le polyester polyol biosourcé (BASAB) a été obtenu à partir du sorbitol selon un procédé d'estérification décrit dans la demande de brevet FR 16/01253. Les propriétés des polyols pétrosourcé et biosourcé sont résumées dans le Tableau 1. Le D-sorbitol commercialisé par TEREOS SYRAL (sorbitol sup. 98%, eau inf. 0,5%, sucres réducteurs inf. 0,1%), le 1,4 butanediol (99%) est commercialisés par la société SIGMA ALDRICH, l'acide adipique (99%) commercialisé par la société ACROS ORGANICS. Le polyisocyanate est le 4,4'-méthylènebis (isocyanate de phényle) (MDI) polymère et la N, N-diméthylcyclohexylamine (DMCHA, catalyseur) provient de BORSODCHEM (Ongronat 2500). Divers catalyseurs bruts tels que 1,3,5-tris (3- [diméthylamino] propyl) -hexahydro-s-triazine fournit par EVONIK (Tegoamin C41), bis (2-diméthylaminoéthyl) éther de BASF (Lupragen N205), 15% en poids. Solution d'octoate de potassium (Ko) et 40% en poids. Un carboxylate de potassium dans l'éthylène glycol (Pc) de EVONIK a été utilisé. L'ignifugeant utilisé est le phosphate de Tris (1-chloro-2-propyle) (TCPP) de SHEKOY, le tensioactif est le polydiméthylsiloxane (B84501) de chez EVONIK et l'éthylène glycol (EG) a été obtenu chez ALFA AESAR (pureté 99%). L'isopentane d'INVENTEC a été utilisé comme agent gonflant. Tous ces produits chimiques ont été utilisés comme reçus sans autre purification.

Tableau 1 : Propriétés principales du polyol polyester pétrosourcé et du BASAB

| | Indice hydroxyle (mg KOH/g) | Indice d'acide (mg KOH/g) | Viscosité (25 °C, mPa.s) | Hydroxyl primaire | Hydroxyl secondaire | Tension de surface (mN/m) |
|---|---|---|---|---|---|---|
| Pétrosourcé | 230-250 | 1.9-2.5 | 4000 | 2 | 0 | 33.6 ± 0.9 |
| BASAB | 490-510 | Inf. 3 | 14 000 | 2 | 4 | 40 ± 0.8 |

**b. Méthode générale d'obtention du BASAB**

La réaction est effectuée dans un réacteur étanche en Inox équipé d'une pâle d'agitation en U, d'un Dean Stark présentant une sortie en haut du condensateur pour pouvoir y lier une pompe à vide et d'une sortie basse pour récupérer les condensats, d'une entrée et d'une sortie de gaz inerte. Dans le réacteur, sont introduit à l'état de poudre du sorbitol et de l'acide adipique dans un ratio molaire 1/2 (sorbitol/acide adipique). Le réacteur est mis sous atmosphère inerte puis est lancé en chauffe. Lorsque la température atteint 100°C, l'agitation est progressivement lancée jusqu'à 170rpm. Quand la température atteint 150°C, la réaction est lancée et poursuivie durant 3h. Au bout de 3h, du 1,4-butanediol (appelé diol par la suite) est introduit dans le réacteur dans un ratio molaire (1,4 butanediol/sorbitol) 2,2/1. La température du milieu réactionnel revient à 150°C (agitation toujours maintenue à 170rpm, atmosphère inerte). 2h30 après le retour à 150°C un passage sous vide partiel est effectué pendant une durée d'une minute puis la pression atmosphérique est ramenée sous atmosphère inerte. 4h30 après l'ajout de diols, un nouveau flush de vide partiel est effectué pendant 2 minutes puis la pression atmosphérique est ramenée sous atmosphère inerte. 6h15 minutes après l'introduction du diol (soit un temps total de réaction de 9h15 min à 150°C), le réacteur est arrêté et le produit de réaction est récupéré à chaud afin d'avoir un minimum de perte lors du transfert de matière du réacteur vers le conditionnement du produit.

**c. Méthode générale de préparation de mousses PUIR**

Le rapport molaire isocyanate / hydroxyle (NCO / OH) a été maintenu à 3,2 dans toutes les formulations PUIR. Pour déterminer la quantité d'isocyanate, tous les groupes hydroxyles réactifs sont pris en compte, c'est-à-dire les polyols, l'eau et les solvants provenant du lot de catalyseurs choisi. Sur la base du procédé de moussage à deux composants, on prépare un premier mélange contenant : des polyols, des catalyseurs, des tensioactifs (polydiméthylsiloxane, B84501), des retardateurs de flamme (TCPP), un agent gonflant (isopentane) et de l'eau. Dans chaque préparation, le nombre de parts (p) de l'eau, le TCPP, les tensioactifs sont constants à 0,9 p, 15 p, 2,5 p, respectivement, et la quantité totale de polyol ne dépasse jamais 100 p. La quantité d'agent gonflant a été maintenue constante à 24 p pour obtenir des mousses de densités comparables. Le mélange a été agité mécaniquement jusqu'à obtenir une fine émulsion blanche avec une incorporation totale de l'agent gonflant. Le mélange et la température des polyisocyanates ont été contrôlés et ajustés à 20°C. Ensuite, la quantité appropriée de polyisocyanate permettant un ratio NCO/OH de 3,2 a été rapidement ajoutée avec une seringue à l'émulsion. Le mélange réactionnel entier a été agité vigoureusement pendant 5 s, puis la mousse mise à expanser librement dans un bêcher jetable de 250 mL à température ambiante (contrôlé à 20°C) ou dans un dispositif FOAMAT. Les constantes caractéristiques de la cinétique de moussage ont été relevées à savoir le temps de crème, le temps de fil et le temps de hors poisse. Avant l'analyse, les échantillons de mousse ont été conservés à température ambiante pendant trois jours pour obtenir une stabilité dimensionnelle complète (absence de retrait).

Une formulation ne contenant uniquement qu'un polyol polyester pétrosourcé a été considérée comme formulation de référence (Tableau 1). Cette formulation est transposée à des formulations contenant 65% et 100% (équiv. à 65p et 100p, respectivement) de BASAB notée 35/65 et 0/100 (PS2412/BASAB), respectivement. Puis la formulation a été optimisée dans des formulations contenant 85% (équiv. à 85 p) de BASAB. Les formulations contenant du BASAB sont présentées dans le Tableau 3.

Tableau 2 : Formulation des mousses PUIR exprimées en nombre de parts

| | | Number of parts. |
|---|---|---|
| Polyols | Pétrosourcé | 100 |
| Catalyseur | C41 | 0.3 |
| | N205 | 0.12 |
| | K0 | 3 |

(suite)

| | | Number of parts. |
|---|---|---|
| Autres | Eau | 1 |
| | Surfactant | 2.5 |
| | Ignifugeant | 15 |

## d. Caractérisations

Les analyses thermogravimétriques (TGA) ont été effectuées à l'aide d'un instrument TA Hi-Res TGA Q5000 sous air reconstitué (débit 25 mL/min). Des échantillons de 1 à 3 mg ont été chauffés de la température ambiante à 700°C (10°C / min). Les principales températures de dégradation caractéristiques sont celles au maximum de la courbe dérivée de perte de poids (DTG) ($T_{deg,\ max}$) et des températures caractéristiques correspondant à 50% ($T_{deg50\%}$) et 100% ($T_{deg100\%}$) de perte de poids ont été rapportées.

La spectroscopie infrarouge a été réalisée avec un spectromètre infrarouge a transformé de Fourier Nicolet 380 utilisé en mode réflexion équipé d'un module diamant ATR (FTIR-ATR). Un fond atmosphérique a été recueilli avant chaque analyse d'échantillon (64 scans, résolution 4 $cm^{-1}$). Tous les spectres ont été normalisés sur un pic d'étirage C-H à 2950 $cm^{-1}$.

La température des mousses, les hauteurs et vitesses d'expansion, la densité et la pression ont été enregistrés à l'aide d'un FOAMAT FPM 150 (Messtechnik GmbH) équipé de récipients cylindriques, 180 mm de hauteur et 150 mm de diamètre, une sonde à ultrasons LR 2-40 PFT enregistrant des hauteurs de mousse, un NiCr / Thermocouple Ni type K et un capteur de pression FPM 150. Les données ont été enregistrées et analysées avec un logiciel spécifique.

Le contenu de cellules fermées est déterminé à l'aide d'un Ultrapyc 1200e de Quantachrome Instruments basé sur la technique de l'expansion du gaz (loi de Boyle). Des échantillons cubiques de mousses (environ 2,5 cm × 2,5 cm × 2,5 cm) sont coupées pour la première mesure, puis l'échantillon a été sectionné une fois de plus en huit pièces et la mesure a été répétée. La deuxième étape permet de corriger le contenu des cellules fermées en fonction de cellules qui ont été endommagées en raison de la coupe de l'échantillon. Les mesures ont été effectuées selon les normes EN IS04590 et ASTM 6226.

La morphologie des cellules des mousses a été observée avec un microscope électronique à balayage à émission électronique (MEB) Jeol JSM-IT100. Les échantillons de mousse cubique ont été coupés avec une lame de microtome et ont été analysés dans deux orientations caractéristiques : longitudinal et transversal à la direction des montées de mousse. En utilisant le logiciel ImageJ (programme de traitement Open Source), la taille moyenne de la cellule a été mesurée comme le rapport d'aspect de la cellule défini par eq.1 22.

$$R = \frac{1}{n}\sum_{i=1}^{n}\frac{D_F^{max}}{D_F^{min}}$$

Où $D_{Fmax}$ et $D_{Fmin}$ sont des diamètres maximum et minimal de Féret, n est le nombre de cellules mesurées pour un échantillon donné.

## II. Résultats et discussion

### a. Cinétique de la mousse de référence PUIR

La mousse PUIR pétrosourcé (100/0) de référence présente un moussage rapide avec des temps caractéristiques présentés dans le Tableau 3.

Afin d'évaluer la cinétique de moussage de ces différentes formulations, ont été mesurés les temps caractéristiques de crème, temps de fil et temps de hors poisse.

Le temps de crème représente l'initiation de la réaction de polyaddition entre les fonctions isocyanates apportées par les polyisocyanate et l'eau ou les groupements alcool apportées par l'ensemble des polyols, co-polyols ou additifs présents dans la formulation. Le temps de crème est caractérisé par un changement de couleur du milieu réactionnel avant l'expansion de la mousse.

Le temps de fil représente le début de la formation du réseau polymère de polyuréthane et/ou polyisocyanurate. Il est caractérisé par la formation de fil collant lorsqu'un contact physique est réalisé avec la mousse en expansion.

Le temps hors poisse représente la fin de la polymérisation du réseau polyuréthane et/ou polyisocyanurate en surface de la mousse. Il est caractérisé par une mousse qui n'est plus collante au touché.

Les temps caractéristiques enregistrés pour 100/0 sont de 10 s, 60 s et 148 s pour le temps de crème, le temps de fil et le temps de hors poisse, respectivement. L'aspect macroscopique d'une telle mousse PUIR est caractéristique (Figure 3a) et présente un col typique induit par la deuxième croissance de la mousse lors de la trimérisation des isocyanates. Cette deuxième croissance est vraiment visible sur les mesures de Foamat présentées à la Figure 1b. Le taux d'expansion de la mousse commence à diminuer après 30 s de réaction et augmente à nouveau après 60 s de réaction. La courbe de température de mousse (Figure 1a) montre également un point d'inflexion à 50 s et augmente jusqu'à 150°C, ce qui correspond à la trimérisation des isocyanates. Le même phénomène est visible sur la Figure 1c, représentant la hauteur maximale de la mousse. Après 50 s, une variation de la pente est observée et la hauteur maximale augmente rapidement de 80 à 100% avec la trimérisation des isocyanates.

**b. Cinétique des mousses PUIR contenant du BASAB**

Deux formulations similaires à la référence (100/0) contenant uniquement du PS 2412 ont été réalisées avec les ratios de polyesters polyol suivants : 35/65 et 0/100 (parts / parts) de PS 2412 / BASAB (Tableau 3). L'analyse du temps de crème des formulations 35/65 et 0/100 permet de constater que le commencement de la réaction de polyaddition entre les polyols et les polyisocyanates est décalé par rapport à la référence de 9 et 14 s respectivement. Le temps de fil de la mousse 0/100 n'est pas mesurable car certainement confondu avec le temps de hors poisse supérieur à 300s alors que la référence présente un temps de hors poisse de 148 s. La comparaison de ces deux temps de hors poisse montre clairement que le mix de catalyseurs traditionnellement utilisés pour la formulation contenant 100% de polyol pétrosourcé n'est pas aussi adapté pour la formulation contenant 100% de polyol biosourcé de l'invention. C'est à dire que l'exothermicité de la réaction de polyaddition est différente et entraîne une plus faible activité des catalyseurs. L'activation de ces catalyseurs est à l'origine de la rapidité de formation du réseau polyuréthane, et à l'origine de la réaction de formation des anneaux triisocyanuriques.

De manière surprenante, la formulation 35/65 présente un temps de hors poisse plus court de 48 s que la référence. Cela signifie que les 35 parts de PS 2412 suffisent à maintenir l'activation du jeu traditionnel de catalyseur. Également, la fonctionnalité supérieure et non conventionnelle du BASAB pour une formulation PUIR permet d'atteindre le temps caractéristique de hors poisse de la mousse plus rapidement.

Deux formulations identiques au témoin ont été préparées en remplaçant le polyol de référence PS 2412 par du BASAB. Des mousses ont pu être obtenues avec de bonnes caractéristiques pour la mousse 35/65. Néanmoins, lors d'un remplacement total, les mousses 0/100 obtenues présentent des temps caractéristiques de moussage mesurés relativement lent.

En effet, de meilleurs résultats sont obtenus en utilisant une combinaison d'un co-polyol avec BASAB. L'EG a été préféré car étant un diol court, il s'est montré très réactif, pour favoriser l'initiation de la première réaction exothermique et augmenter la réaction entre le BASAB et les molécules de polyisocyanate. Après différent essais de ratio BASAB/EG le rapport de 85%pds/15%pds a montré le meilleur résultat dans la formulation de mousse PUIR.

Deux catalyseurs ont été comparés : l'octoate de potassium (Ko) et un carboxylate de potassium (Pc). Ce dernier catalyseur plus petit, présente une plus grande mobilité et donc une activité plus importante dans le milieu.

Les formulations optimisées résultantes à savoir une formulation B1-K0 comprenant 100% de polyol polyester biosourcé (85 parts de BASAB et 15 parts d'éthylène glycol) et de l'octoate de potassium (Ko) et une formulation B2-PC comprenant 100% de polyol polyester biosourcé (85 parts de BASAB et 15 parts d'éthylène glycol et du carboxylate de potassium (Pc) sont détaillées dans le Tableau 3. La formulation de référence (100/0) comprend 100% de polyol polyester pétrosourcé (PS 2412) et de l'octoate de potassium (Ko).

Tableau 3 : Taux de catalyseur et temps caractéristiques des différentes mousses PUIR.[a]: exprimé en nombre de parts vis à vis du produit final, [b]: exprimé en pourcentage par rapport au produit final, n.m: non mesurable.

| | | **Reference (100/0)** | **35/65** | **0/100** | **B1-K0** | **B2-PC** |
|---|---|---|---|---|---|---|
| Formulati on | PS 2412 | 100[a] | 35 | 0 | 0[a] | 0[a] |
| | BASAB | 0[a] | 65 | 100 | 85[a] | 85 |
| | EG | 0[a] | 0 | 0 | 15[a] | 15[a] |
| | Ko | 0.12[b] | 0.12[b] | 0.12[b] | 0.17[b] | 0[b] |
| | N205 | 0.03 [b] | 0.03[b] | 0.03[b] | 0.08 [b] | 0.22[b] |
| | Tegoamine C41 | 0.08[b] | 0.08[b] | 0.08[b] | 0.21[b] | 0.11[b] |
| | Pc | 0[b] | 0[b] | 0[b] | 0[b] | 0.97[b] |
| Temps caractéris tiques | Temps de crème (s) | 10 | 19 | 24 | 12 | 11 |
| | Temps de fil (s) | 60 | 76 | n.m | 134 | 82 |
| | Temps de hors poisse (s) | 148 | 100 | ≥ 300 | 166 | 120 |

La formulation B1-KO catalysée avec le même catalyseur que la référence mais en quantité supérieure présente un temps de crème relativement similaire à cette dernière (Tableau 3). Cependant, B1-KO présente un temps de fil ayant un délai de 74 s comparativement à celui de la référence et un délai de 18 s pour le temps de hors poisse. Il ressort donc de ces résultats que la formulation B1-K0 présente des caractéristiques distinctes en termes de temps caractéristique comparativement à la référence. Ces différences, notamment l'allongement des temps caractéristiques constitue un avantage à la formulation de mousse PUIR rigide en bloc élaboré dans des moules.

D'autre part, comparativement à la référence, la formulation biosourcée B2-PC présente un catalyseur différent de celui de la référence. On observe que le temps de crème de cette formulation ainsi que les temps de fil sont plus proches des temps de la formulation de référence alors que le temps de hors poisse de B2-PC est plus rapide de 28 s par rapport à celui de la référence. Il ressort donc de ces résultats que la formulation B2-PC présente des caractéristiques distinctes en termes de temps caractéristique comparativement à la B1-K0. Cette formulation ayant des temps caractéristiques plus courts et similaires à ceux de la référence constitue un avantage pour les procédés de production en ligne de panneau d'isolation de mousse PUIR rigide.

Au-delà des temps caractéristiques, la différence la plus importante entre ces formulations est macroscopique.

En effet, les caractéristiques des mousses des formulations précédentes ont été comparées. Il en ressort que la mousse B2-PC présente une surface propre, avec une peau extérieure lisse, similaire à la référence, tandis que B1-K0 présente une surface irrégulière (Figure 3b et c) (présence de fissures et de bulles).

L'hypothèse principale justifiant ces différences de surface entre la mousse B1-K0 d'une part et les mousses B2-PC et de référence d'autre part est basée sur les différences de temps de fil. En effet, le temps de fil de 134 s de B1-KO est plus long que celui de B2-PC qui est de 82 s.

Étant donné que les formulations B1-K0 et B2-PC contiennent le même polymère, le temps de fil plus long traduit un temps plus long pour atteindre le même degré de polymérisation et donc une instabilité ou une fragilité du matériau au court de cette étape de polymérisation. Cette fragilité fait que les parois des cellules s'effondrent sous la pression du gaz en expansion générant des fissures et des bulles visibles sur la surface de la mousse. Cela a lieu avant le durcissement de la mousse et la fin de la polymérisation, soit avant la fin du processus de moussage.

L'évolution de la température interne des mousses pendant le procédé de moussage a été évaluée (Figure 2). Il apparait clairement que la mousse B1-PC monte plus rapidement en température que la mousse B1-K0. Cela reflète une plus grande réactivité du milieu réactionnel, certainement dû aux choix de catalyseurs. En outre, l'échantillon B1-KO présente une température de moussage inférieure en tout point aux deux autres mousses présentées. La courbe de température de moussage de B1-PC présente un profil similaire à celle de la référence jusqu'à l'inflexion caractéristique de celle-ci correspondant à la trimérisation des isocyanates.

Enfin, on constate que la cinétique globale de la mousse PUIR de B2-PC est très proche de la mousse de référence et la température de la mousse est de 140°C, ce qui est similaire à la température de moussage de la référence

pétrosourcée.

La meilleure réactivité de moussage et donc la meilleure cinétique de moussage, a été obtenue par l'augmentation de la température de moussage (notamment par le changement de catalyseur), par l'augmentation de la quantité de BASAB dans le mélange et par l'ajout d'un co-polyol.

Ainsi, ces résultats démontrent qu'une formulation de mousse PUIR comprenant des polyol polyesters biosourcés et présentant des caractéristiques comparables à celles d'une formulation à base de polyol polyesters pétrosourcé peut être obtenue. Une telle formulation est particulièrement avantageuse pour une production rapide en continue sur ligne de blocs ou de panneaux de mousse. D'autre part, ces résultats révèlent également que d'autres types de formulations PUIR comprenant des polyol polyesters biosourcés et présentant des caractéristiques de moussage plus lentes que la référence à base de polyol polyesters pétrosourcés peuvent également être obtenues. De telles formulations représentent un avantage pour la production de mousse en bloc moulé. Le polyol polyester biosourcé (BASAB) est particulièrement avantageux en ce qu'il offre l'opportunité d'adapter les caractéristiques de moussage ou la cinétique de la mousse en fonction des applications ou des procédés de fabrications souhaités.

**c. Taux de cellules fermées et morphologie des mousses**

Les morphologies des mousses obtenues ont été comparées par MEB. La Figure 4 montre les images MEB d'échantillons de la mousse PUIR de référence découpés dans la direction transversale et longitudinale à la montée de la mousse après moussage. Une structure typique en nid d'abeilles dans la direction transversale est clairement observée. L'étirement de la cellule dans la direction longitudinale est caractéristique d'un procédé de moussage partiellement libre réalisé dans un conteneur cylindrique ouvert (M. C. Hawkins, J. Cell. Plast., 2005, 41, 267-285). Les observations en MEB ont permis la mesure des coefficients anisotropes R des mousses PUIR : 100/0, 35/65, 0/100, B1-KO B2-PC étudiées (Tableau 4).

Les coefficients anisotropes (R) reflètent la forme des cellules d'une mousse. Le coefficient R est le rapport des deux diamètres maximaux mesurables dans une cellule. Ainsi une cellule parfaitement ronde présentera un coefficient R égale à 1 (tous les diamètres sont identiques dans un cercle). À l'opposé une cellule étirée et de forme ovale présentera un coefficient R supérieur à 1. Dans la présente étude les coefficients R sont déterminés dans deux plans différents. Cela permet d'évaluer la forme des cellules dans une coupe transverse au sens d'expansion des mousses PUIR et de même dans le sens longitudinal à l'expansion des mousses PUIR.

On observe que pour les coefficients R des formulations 100/0, B1-KO et B2-PC sont proches de 1,8 dans la direction longitudinale. Ceci traduit une forme ovale des cellules de la mousse. Dans la direction transversale à la montée de la mousse, le coefficient R calculé est plus proche de 1,2. Il reflète ici une forme de cellules plus proche de la forme sphérique. Les formulations 35/65 et 0/100 présentent des coefficients R moins comparables. La large distribution des tailles de cellules de ces mousses qui se traduit par des écart-types importants de l'ensemble de leurs diamètres de Féret dans les directions longitudinale et transversale engendre des cellules de forme très anisotrope.

Si l'on compare la mousse de référence aux mousses 34/65 et 0/100, ces dernières présentent des cellules environ 2 à 4 fois plus grandes que la référence à base de polyol 100% pétrosourcé dans l'ensemble des directions d'études. La taille des cellules est un critère impactant pour les propriétés finales d'une mousse PUIR. Par exemple, de grandes cellules engendrent de moins bonnes propriétés d'isolation thermique.

Si l'on compare la mousse de référence aux mousses biosourcées PUIR B1-KO et B2-PC, ces dernières présentent des cellules de tailles presque similaires à celles de la référence dans l'ensemble des directions (Tableau 4). Par rapport aux formulations 35/65 et 0/100 leurs tailles de cellule sont nettement inférieures. Ce gain majeur sur les formulations biosourcées est un avantage pour leur utilisation dans le domaine de l'isolation thermique du bâtiment par exemple.

Tableau 4 : Diamètre de Féret et coefficient d'anisotropie (R) de l'ensemble des mousses PUIR dans les directions longitudinales et transversales au sens d'expansion des mousses

| | | **100/0** | **35/65** | **0/100** | **B1-K0** | **B2-PC** |
|---|---|---|---|---|---|---|
| Direction longitudinale | Féret max, $D_F^{max}$ (μm) | 408 ± 117 | 643 ± 189 | 860 ± 170 | 524 ± 215 | 521 ± 123 |
| | Féret min, $D_F^{min}$ (μm) | 223 ±4 | 518 ± 147 | 550 ± 130 | 295 ± 112 | 298 ± 67 |
| | R= $D_F^{max}$ / $D_F^{min}$ | 1,83 | 1,25 | 1,56 | 1,78 | 1,75 |

(suite)

| | | 100/0 | 35/65 | 0/100 | B1-K0 | B2-PC |
|---|---|---|---|---|---|---|
| Direction transversale | Féret max, $D_F^{max}$ ($\mu$m) | 275 $\pm$ 72 | 940 $\pm$ 260 | 1240 $\pm$ 380 | 386 $\pm$ 123 | 448 $\pm$ 122 |
| | Féret min, $D_F^{min}$ ($\mu$m) | 242 $\pm$ 72 | 420 $\pm$ 90 | 990 $\pm$ 300 | 324 $\pm$ 116 | 347 $\pm$ 122 |
| | R= $D_F^{max}$ / $D_F^{min}$ | 1,14 | 2,24 | 1,25 | 1,19 | 1,29 |

L'étude des profils cinétiques de moussage précédente a montré que les températures atteintes par le milieu réactionnel pendant le procédé de moussage sont plus basses pour les mousses B1-K0 et B2-PC. Ces basses températures sont responsables d'un retard avant la trimérisation des isocyanates, à l'origine des taux de réaction plus bas (temps de fil plus long). L'augmentation du temps de fil induit une augmentation du processus de coalescence des bulles de gaz avant la polymérisation complète du réseau de polyuréthane et de polyisocyanurate de la mousse, ce qui explique l'observation de tailles de cellules plus importantes pour les mousses biosourcées.

**d. Propriétés des mousses : densité, taux de cellules fermées et composition chimique (FT-IR)**

Tableau 5 : Propriété des mousses PUIR. n.d. : non déterminé

| | 100/0 | 35/65 | 0/100 | B1-K0 | B2-PC |
|---|---|---|---|---|---|
| Densité apparente (kg/m$^3$) | 31.1 | 39.8 | n.d. | 33.8 $\pm$ 2 | 32.8 $\pm$ 0.8 |
| Taux de cellules fermées (%) | 95 | < 50 | < 50 | 86 | 85 |

La densité apparente des mousses présentées dans le Tableau 5 est similaire pour toutes les formulations PUIR, et est comprise entre 31 et 40 kg/m$^3$. La mousse 35/65 présente la plus haute densité apparente (39.8 kg/m$^3$) et c'est également la mousse qui présente la température de moussage la plus basse. La faible température de moussage a limité l'expansion de l'agent gonflant, conduisant à une mousse légèrement plus dense que les autres. Les mousses B1-K0 et B2-PC, a la formulation optimisée ne présente pas cette caractéristique puisque leur densité est plus proche de celle de la référence.

Afin de confirmer la nature chimique des mousses obtenues, une analyse par spectrométrie infrarouge (FT-IR) a été effectuée. Les spectres FT-IR des mousses formulées sont présentés dans la Figure 5. Toutes les mousses présentent des pics caractéristiques comme les vibrations d'étirement des groupes N-H, à 3400-3200 cm$^{-1}$ et les vibrations d'étirement de la liaison C = O présente dans les groupements uréthanes à 1705 cm$^{-1}$. Les signaux situés à 2955 cm$^{-1}$ et 2276 cm$^{-1}$ sont respectivement attribués à l'étirage de liaison C-H du squelette de polyuréthane et aux groupes NCO résiduels n'ayant pas réagi. Le signal à 1596 cm$^{-1}$ correspond à l'étirage Ar-H dans des groupes phényle provenant du polyisocyanate polymérique. Le signal de flexion des groupes N-H est situé à 1509 cm$^{-1}$ et l'étirement C-O à 1220 cm$^{-1}$. Ensuite, un signal fort à 1408 cm$^{-1}$ est attribué à la présence d'anneaux d'isocyanurate typiques de la formulation de mousse PUIR.

On en conclu donc que les mousses obtenues et notamment les mousses à base de polyol polyester biosourcé présentent une composition chimique similaire à celle de la mousse à base de 100% de polyol polyester pétrosourcé. Cela prouve que les différences concernant les temps caractéristiques ou températures de moussage précédemment observées n'ont pas empêché la bonne formation d'un réseau PUIR dans l'ensemble des formulations.

**e. Résistance thermique des mousses**

La stabilité thermique des échantillons de mousses PUIR a été étudiée par analyse thermogravimétrique des courbes ATG et DTGA de toutes les mousses PUIR (non montrées). Toutes les mousses PUIR présentent une perte de poids classique en deux étapes. Les mousses PUIR B1-K0 et B2-PC présentent une stabilité thermique supérieure par rapport à la référence. Le Tableau 6 montre les températures au maximum de la courbe dérivée de la perte de poids : $T_{deg\ max1}$ et $T_{deg\ max2}$. $T_{deg\ max1}$ se situe aux alentours de 300 ° C pour les trois mousses. $T_{deg\ max2}$ est observée à une température de 523 ° C pour la référence alors que les mousse B1-K0 et B1-PC présente des $T_{deg\ max2}$ plus élevée, respectivement 538 et 534 °C.

De plus, elles présentent un épaulement de la courbe DTGA à plus de 600 °C. La première $T_{deg\ max1}$ correspond à la décomposition de la liaison uréthane. Le mécanisme de décomposition de la liaison uréthane est généralement décrit comme une dissociation simultanée de l'isocyanate et de l'alcool, la formation d'une amine primaire et secondaire et la formation d'oléfines. La deuxième $T_{deg\ max2}$ est plus prononcée que la première $T_{deg\ max1}$ et est associée à la double dégradation de l'isocyanurate et du clivage des liaisons carbone-carbone (J. E. Sheridan and C. A. Haines, J. Cell. Plast., 1971, 7, 135-139). La première perte de poids est moins importante car il existe une liaison isocyanurate. Les isocyanurates sont thermiquement plus stables que l'uréthane en raison de l'absence d'hydrogène labile et donc la seconde perte de poids est principalement causée par le clivage carbone-carbone (H. E. Reymore et al.,n J. Cell. Plast., 1975, 11, 328-344). Dans le cas spécifique de B1-KO et B2-PC, $T_{deg\ max2}$ est supérieur et est attribué à leur concentration plus élevée en BASAB par rapport à la référence. La valeur OH plus élevée du BASAB par rapport au PS 2412 augmente la formation de liaisons uréthanes et la réticulation du réseau PUIR (A. A. Septevani, et al Ind. Crops Prod., 2015, 66, 16-26 *;* I. Javni, Z. S. et al., J. Appl. Polym. Sci., 2000, 77, 1723-1734*)* le rendant plus résistant à la dégradation thermique.

Le Tableau 6 présente également deux températures correspondant à 50% ($T_{deg\ 50\%}$) et 100% ($T_{deg\ 100\%}$) de perte de poids des mousses PUIR, respectivement $T_{deg\ 50\%}$ et $T_{deg\ 100\%}$. Ces dernières sont similaires entre les mousses de référence et B1-K0. L'échantillon B2-PC présente une $T_{deg\ 50\%}$ et une $T_{deg\ 100\%}$ supérieures à celles de la mousse de référence ce qui est en accord avec les observations précédentes. Par conséquent, la formulation B2-PC permet l'obtention d'une mousse plus résistante à la température que la mousse de référence à base de polyol polyester pétrosourcé.

Tableau 6: Degradation temperature at 95% and 50% weight loss of PUIR foams samples

| Sample | ATG | | DTG | |
|---|---|---|---|---|
| | $T_{deg50\%}$ (°C) | $T_{deg100\%}$ (°C) | $T_{deg\ max1}$ | $T_{deg\ max2}$ |
| 0% (Reference) | 448 | 645 | 301 | 523 |
| B1-K0-PC | 458 | 632 | 300 | 538 |
| B2-PC | 499 | 690 | 295 | 534 |

Des mousses PUIR à cellules fermées basées sur la substitution totale d'un polyester polyol pétrosourcé par le polyester polyol biosourcé ont été préparées avec succès. L'optimisation de la formulation à permit d'obtenir une cinétique de moussage similaire à celle de la référence pétrosourcée. L'étude a été menée par l'utilisation de deux catalyseurs différents. Les mousses PUIR présentent une teneur élevée en cellules fermées ce qui très intéressant pour répondre à des caractéristiques d'isolation thermique. Enfin le point le plus marquant concerne les mousses PUIR biosourcées qui présentent une stabilité à la dégradation thermique supérieure à celle de la référence pétrosourcée.

## Revendications

1. Mousse rigide ou une composition permettant l'obtention d'une mousse rigide comprenant un polyol polyester ou un polymère comprenant un polyol polyester, ledit polyol polyester étant de formule générale **Rx-Ry-Z-Ry'-Rx'** dans laquelle

   - **Z** est un sucre alcool en C3 à C8,
   - **Ry** et **Ry'** sont des diesters de formule $-OOC-C_n-COO-$ avec **n** compris entre 2 et 34, et
   - **Rx** et **Rx'** sont des monoalcools, identiques ou différents en C2 à C12.

2. Mousse rigide ou composition permettant l'obtention d'une mousse rigide selon la revendication **1**, dans laquelle **Z** est un sucre alcool en C4 à C7, typiquement en C5-C6.

3. Mousse rigide ou composition permettant l'obtention d'une mousse rigide selon la revendication **1** ou la revendication **2,** dans laquelle **n** est compris entre 3 et 22, typiquement entre 4 et 10.

4. Mousse rigide ou composition permettant l'obtention d'une mousse rigide selon l'une quelconque des revendications **1** à **3,** dans laquelle **Rx** et **Rx'** sont des monoalcools en C3 à C8, typiquement en C4.

5. Mousse rigide ou composition permettant l'obtention d'une mousse rigide selon l'une quelconque des revendications

**1** à **4, caractérisé en ce que** le sucre alcool **Z** est choisi parmi le glycérol, le sorbitol, l'érythritol, le xylitol, l'arabitol, le ribitol, le dulcitol, le mannitol et le volémitol.

6. Mousse rigide ou composition permettant l'obtention d'une mousse rigide selon l'une quelconque des revendications **1** à **5, caractérisé en ce que Ry** et **Ry'** sont les diesters correspondant à deux diacides Y et Y' indépendamment choisis parmi l'acide butanedioïque, l'acide pentanedioïque, l'acide hexanedioïque, l'acide heptanedioïque, l'acide octanedioïque, l'acide nonanedioïque, l'acide décanedioïque, l'acide undécanedioïque, l'acide dodécanedioïque, l'acide tridécanedioïque, l'acide tétradécanedioïque, l'acide pentadécanedioïque, l'acide hexadécanedioïque et leur mélange.

7. Mousse rigide ou composition permettant l'obtention d'une mousse rigide selon l'une quelconque des revendications **1** à **6, caractérisé en ce que Rx** et **Rx'** sont les monoalcools correspondant à deux diols X et X' indépendamment choisis parmi le 1,2-éthanediol, le 1,3-propanediol, le 1,4-butanediol, le 1,6-hexanediol, le 1,8-octanediol, le 1,10-décanediol, le 1,12-dodécanediol et leur mélange.

8. Mousse rigide ou composition permettant l'obtention d'une mousse rigide selon l'une quelconque des revendications **1** à **7,** comprenant au moins un catalyseur de réaction, au moins un agent gonflant, un stabilisant, au moins un polyisocyanate ayant une fonctionnalité au moins égale à 2 et, optionnellement, au moins un co-polyol.

9. Mousse rigide ou composition permettant l'obtention d'une mousse rigide selon l'une quelconque des revendications **1** à **8, caractérisée en ce qu'**elle comprend au moins un co-polyol en C2 à C8.

10. Mousse rigide ou composition permettant l'obtention d'une mousse rigide selon la revendication **8** ou la revendication **9, caractérisée en ce qu'**elle présente un ratio polyol polyester/co-polyol(s) de 70/30 à 99/1 en nombre de parts, préférentiellement de 75/25 à 95/5 en nombre de parts.

11. Mousse rigide ou composition permettant l'obtention d'une mousse rigide selon l'une quelconque des revendications **8** à **10**, **caractérisée en ce que** l'au moins un co-polyol est choisi parmi l'éthylène glycol, le glycérol, le 1,4-butanediol, le butane-1,3-diol, 1,3-propanediol, le propane-1,2-diol, 1,5-pentanediol, 1,6-hexanediol, 1,2-propylene glycol, 3-oxapentane-1,5-diol, 2-[2-(2-hydroxyéthoxy)éthoxy]éthanol, le benzène-1,2,4-triol, le benzène 1,2,3-triol, le benzène 1,3,5-triol, le sorbitol, l'érythritol, le xylitol, l'arabitol, le ribitol, le dulcitol, le mannitol et le volémitol.

12. Mousse rigide ou composition permettant l'obtention d'une mousse rigide selon l'une quelconque des revendications **1** à **11**, **caractérisée en ce qu'**elle comprend

   - au moins 1 à 100 parts d'un polyol polyester selon l'invention,
   - 0 à 70 parts d'au moins un co-polyol, préférentiellement de 1 à 50 parts d'au moins un co-polyol,
   - 150 à 500 parts d'un polyisocyanate,
   - 0,5 à 5 parts d'un catalyseur, typiquement d'un catalyseur aminé,
   - 0,5 à 15 parts d'un agent gonflant, typiquement 0,5 à 12 parts, préférentiellement d'un agent gonflant chimique,
   - 0 à 5 parts d'un stabilisant tel qu'un copolymère polyéther-polysiloxane et
   - 0 à 20 parts d'un agent ignifugeant.

13. Mousse rigide ou composition permettant l'obtention d'une mousse rigide selon l'une quelconque des revendications **1** à **12**, **caractérisée en ce qu'**elle comprend 0,5 à 12 parts d'un agent gonflant chimique tel que l'eau et 0 à 60 parts d'un agent gonflant physique.

14. Panneau ou un bloc de mousse rigide comprenant une mousse rigide selon l'une quelconque des revendications **1** à **13.**

15. Méthode d'isolation thermique, phonique ou cryogénique ou une méthode de comblement, d'étanchéification, de scellage ou d'amélioration de la flottaison d'un bâtiment ou d'un objet par le dépôt ou l'introduction de blocs ou de panneaux de mousse rigide selon la revendication **14** ou par la projection d'une mousse rigide ou d'une composition permettant l'obtention d'une mousse rigide selon l'une quelconque des revendications **1** à **13.**

## Patentansprüche

1. Hartschaum oder Zusammensetzung, die das Erlangen eines Hartschaums ermöglicht, umfassend ein Polyesterpolyol oder ein Polymer, umfassend ein Polyesterpolyol, wobei das Polyesterpolyol die allgemeine Formel **Rx-Ry-Z-Ry'-Rx'** aufweist, wobei

   - **Z** ein C3- bis C8-Zuckeralkohol ist,
   - **Ry** und **Ry'** Diester der Formel -OOC-Cn-COO- sind, wobei **n** zwischen 2 und 34 liegt, und
   - **Rx** und **Rx'** gleiche oder verschiedene C2- bis C12-Monoalkohole sind.

2. Hartschaum oder Zusammensetzung, die das Erlangen eines Hartschaums ermöglicht, nach Anspruch **1**, wobei **Z** ein C4- bis C7-, in der Regel C5-C6-Zuckeralkoholist.

3. Hartschaum oder Zusammensetzung, die das Erlangen eines Hartschaums ermöglicht, nach Anspruch **1** oder Anspruch **2,** wobei **n** zwischen 3 und 22, in der Regel zwischen 4 und 10 liegt.

4. Hartschaum oder Zusammensetzung, die das Erlangen eines Hartschaums ermöglicht, nach einem der Ansprüche **1** bis **3,** wobei **Rx** und **Rx'** C3- bis C8-, in der Regel C4-Monoalkohole sind.

5. Hartschaum oder Zusammensetzung, die das Erlangen eines Hartschaums ermöglicht, nach einem der Ansprüche **1** bis **4, dadurch gekennzeichnet, dass** der Zuckeralkohol **Z** aus Glycerin, Sorbit, Erythrit, Xylit, Arabit, Ribit, Dulcit, Mannit und Volemit ausgewählt ist.

6. Hartschaum oder Zusammensetzung, die das Erlangen eines Hartschaums ermöglicht, nach einem der Ansprüche **1** bis **5, dadurch gekennzeichnet, dass Ry** und **Ry'** Diester sind, die zwei Disäuren Y und Y' entsprechen, die unabhängig aus Butandisäure, Pentandisäure, Hexandisäure, Heptandisäure, Octandisäure, Nonandisäure, Decandisäure, Undecandisäure, Dodecandisäure, Tridecandisäure, Tetradecandisäure, Pentadecandisäure, Hexadecandisäure und deren Gemisch ausgewählt sind.

7. Hartschaum oder Zusammensetzung, die das Erlangen eines Hartschaums ermöglicht, nach einem der Ansprüche **1** bis **6, dadurch gekennzeichnet, dass Rx** und **Rx'** Monoalkohole sind, die zwei Diolen X und X' entsprechen, die unabhängig aus 1,2-Ethandiol, 1,3-Propandiol, 1,4-Butandiol, 1,6-Hexandiol, 1,8-Octandiol, 1,10-Decandiol, 1,12-Dodecandiol und deren Gemisch ausgewählt sind.

8. Hartschaum oder Zusammensetzung, die das Erlangen eines Hartschaums ermöglicht, nach einem der Ansprüche **1** bis **7,** umfassend mindestens einen Reaktionskatalysator, mindestens ein Treibmittel, einen Stabilisator, mindestens einen Polyisocyanat mit einer Funktionalität, die mindestens gleich 2 ist, und optional mindestens einem Copolyol.

9. Hartschaum oder Zusammensetzung, die das Erlangen eines Hartschaums ermöglicht, nach einem der Ansprüche **1** bis **8, dadurch gekennzeichnet, dass** er bzw. sie mindestens ein C2- bis C8-Copolyol umfasst.

10. Hartschaum oder Zusammensetzung, die das Erlangen eines Hartschaums ermöglicht, nach Anspruch **8** oder Anspruch **9, dadurch gekennzeichnet, dass** er bzw. sie ein Verhältnis von Polyesterpolyol:Copolyol(en) von 70:30 bis 99:1 in Anteilen, vorzugsweise von 75:25 bis 95:5 in Anteilen aufweist.

11. Hartschaum oder Zusammensetzung, die das Erlangen eines Hartschaums ermöglicht, nach einem der Ansprüche **8** bis **10**, **dadurch gekennzeichnet, dass** das mindestens eine Copolyol aus Ethylenglykol, Glycerin, 1,4-Butandiol, Butan-1,3-diol, 1,3-Propandiol, Propan-1,2-diol, 1,5-Pentandiol, 1,6-Hexandiol, 1,2-Propylenglykol, 3-Oxapentan-1,5-diol, 2-[2-(2-Hydroxyethoxy)ethoxy]ethanol, Benzol-1,2,4-triol, Benzol-1,2,3-triol, Benzol-1,3,5-triol, Sorbit, Erythrit, Xylit, Arabit, Ribit, Dulcit, Mannit und Volemit ausgewählt ist.

12. Hartschaum oder Zusammensetzung, die das Erlangen eines Hartschaums ermöglicht, nach einem der Ansprüche **1** bis **11, dadurch gekennzeichnet, dass** er bzw. sie umfasst:

   - mindestens 1 bis 100 Teile eines erfindungsgemäßen Polyesterpolyols,
   - 0 bis 70 Teile mindestens eines Copolyols, vorzugsweise von 1 bis 50 Teile mindestens eines Copolyols,
   - 150 bis 500 Teile eines Polyisocyanats,

- 0,5 bis 5 Teile eines Katalysators, in der Regel eines aminierten Katalysators,
- 0,5 bis 15 Teile eines Treibmittels, in der Regel 0,5 bis 12 Teile, vorzugsweise eines chemischen Treibmittels,
- 0 bis 5 Teile eines Stabilisators, wie eines Polyether-Polysiloxan-Copolymers, und
- 0 bis 20 Teile eines Flammschutzmittels.

**13.** Hartschaum oder Zusammensetzung, die das Erlangen eines Hartschaums ermöglicht, nach einem der Ansprüche **1** bis **12**, **dadurch gekennzeichnet, dass** er bzw. sie 0,5 bis 12 Teile eines chemischen Treibmittels, wie Wasser, und 0 bis 60 Teile eines physikalischen Treibmittels umfasst.

**14.** Hartschaumplatte oder -block, umfassend einen Hartschaum nach einem der Ansprüche **1** bis **13.**

**15.** Verfahren zur Wärme-, Schall- oder Kältedämmung oder Verfahren zur Auffüllung, Abdichtung, Versiegelung oder Verbesserung der Wasserlinie eines Gebäudes oder eines Gegenstands durch die Ablagerung oder Einführung von Hartschaumblöcken oder -platten nach Anspruch **14** oder durch die Spritzung eines Hartschaums oder einer Zusammensetzung, die das Erlangen eines Hartschaums ermöglicht, nach einem der Ansprüche **1** bis **13.**

## Claims

**1.** Rigid foam or a composition allowing a rigid foam to be obtained comprising a polyester polyol or a polymer comprising a polyester polyol, said polyester polyol being of general formula **Rx-Ry-Z-Ry'-Rx'** wherein

- **Z** is a C3 to C8 alcohol sugar,
- **Ry** and **Ry'** are diesters of formula -OOC-Cn-COO- with **n** ranging from 2 to 34, and
- **Rx** and **Rx'** are identical or different C2 to C12 monoalcohols.

**2.** Rigid foam or composition allowing a rigid foam to be obtained according to claim **1,** wherein **Z** is a C4 to C7, typically a C5-C6, alcohol sugar.

**3.** Rigid foam or composition allowing a rigid foam to be obtained according to claim **1** or claim **2,** wherein **n** is ranging from 3 to 22, typically from 4 to 10.

**4.** Rigid foam or composition allowing a rigid foam to be obtained according to any one of claims **1** to **3,** wherein **Rx** and **Rx'** are C3 to C8, typically C4, monoalcohols.

**5.** Rigid foam or composition allowing a rigid foam to be obtained according to any one of claims **1** to **4, characterised in that** the alcohol sugar **Z** is chosen from glycerol, sorbitol, erythritol, xylitol, arabitol, ribitol, dulcitol, mannitol and volemitol.

**6.** Rigid foam or composition allowing a rigid foam to be obtained according to any one of claims **1** to **5, characterised in that Ry** and **Ry'** are the diesters corresponding to two diacids Y and Y' independently chosen from butanedioic acid, pentanedioic acid, hexanedioic acid, heptanedioic acid, octanedioic acid, nonanedioic acid, decanedioic acid, undecanedioic acid, dodecanedioic acid, tridecanedioic acid, tetradecanedioic acid, pentadecanedioic acid, hexadecanedioic acid and a mixture thereof.

**7.** Rigid foam or composition allowing a rigid foam to be obtained according to any one of claims **1** to **6, characterised in that Rx** and **Rx'** are the monoalcohols corresponding to two diols X and X' independently chosen from 1,2-ethanediol, 1,3-propanediol, 1,4-butanediol, 1,6-hexanediol, 1,8- octanediol, 1,10-decanediol, 1,12-dodecanediol and a mixture thereof.

**8.** Rigid foam or composition allowing a rigid foam to be obtained according to any one of claims **1** to **7,** comprising at least one reaction catalyst, at least one swelling agent, a stabiliser, at least one polyisocyanate having a functionality at least equal to 2, and, optionally, at least one co-polyol.

**9.** Rigid foam or composition allowing a rigid foam to be obtained according to any one of claims **1** to **8, characterised in that** it comprises at least one C2 to C8 co-polyol.

**10.** Rigid foam or composition allowing a rigid foam to be obtained according to claim **8** or claim **9, characterised in**

**that** it presents a polyester polyol/copolyol(s) ratio from 70/30 to 99/1 in number of parts, preferably from 75/25 to 95/5 in number of parts.

**11.** Rigid foam or composition allowing a rigid foam to be obtained according to any one of claims **8** to **10, characterised in that** the at least one co-polyol is chosen from ethylene glycol, glycerol, 1,4-butanediol, butane-1,3-diol, 1,3-propanediol, propane-1,2-diol, 1,5-pentanediol, 1,6-hexanediol, 1,2-propylene glycol, 3-oxapentane-1,5-diol, 2-[2-(2-hydroxyethoxy)ethoxy]ethanol, benzene-1,2,4-triol, benzene 1,2,3-triol, benzene 1,3,5-triol, sorbitol, erythritol, xylitol, arabitol, ribitol, dulcitol, mannitol and volemitol.

**12.** Rigid foam or composition allowing a rigid foam to be obtained according to any one of claims **1** to **11, characterised in that** it comprises

- at least 1 to 100 parts of a polyester polyol according to the invention,
- 0 to 70 parts of at least one co-polyol, preferably from 1 to 50 parts of at least one co-polyol,
- 150 to 500 parts of a polyisocyanate,
- 0.5 to 5 parts of a catalyst, typically of an amine catalyst,
- 0.5 to 15 parts of a swelling agent, typically 0.5 to 12 parts, preferably of a chemical swelling agent,
- 0 to 5 parts of a stabiliser such as a polyether-polysiloxane copolymer and
- 0 to 20 parts of a flame-proofing agent.

**13.** Rigid foam or composition allowing a rigid foam to be obtained according to any one of claims **1** to **12, characterised in that** it comprises 0.5 to 12 parts of a chemical swelling agent such as water and 0 to 60 parts of a physical swelling agent.

**14.** Panel or block of rigid foam comprising a rigid foam according to any one of claims **1** to **13.**

**15.** Method for thermal, sound, or cryogenic insulation or a method for filling, water-proofing, sealing or improving the buoyancy of a vessel or of an object by the deposition or the introduction of blocks or panels of rigid foam according to claim **14** or by the spraying of a rigid foam or of a composition allowing a rigid foam to be obtained according to any one of claims **1** à **13.**

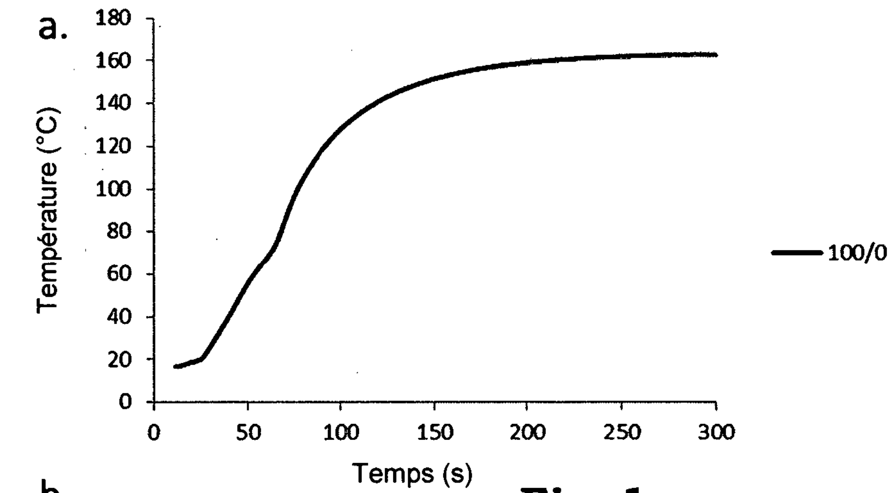


**Fig. 1a**

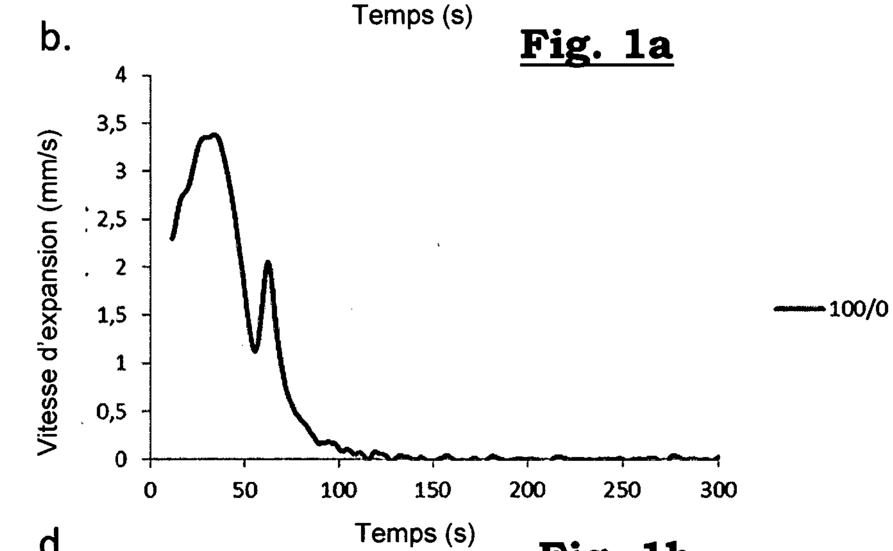


**Fig. 1b**

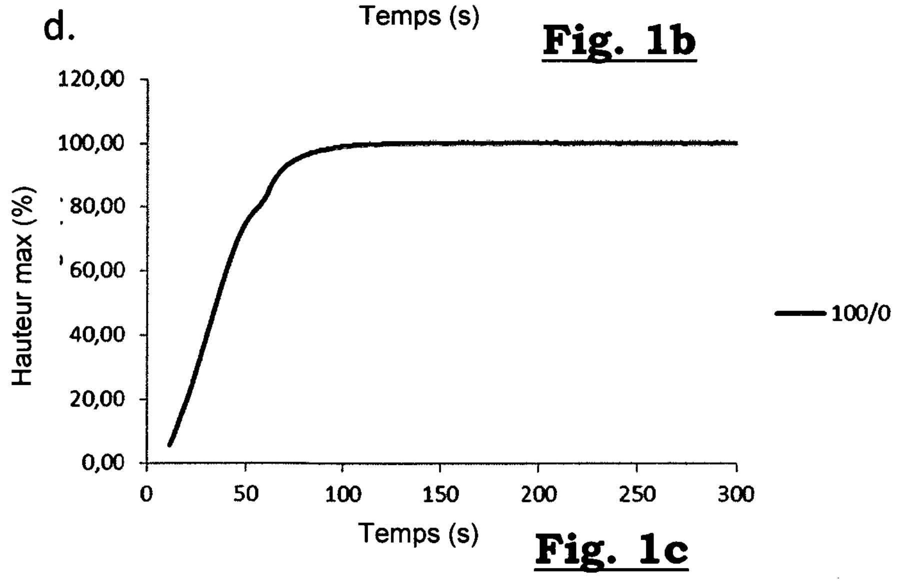


**Fig. 1c**

**Fig. 1**

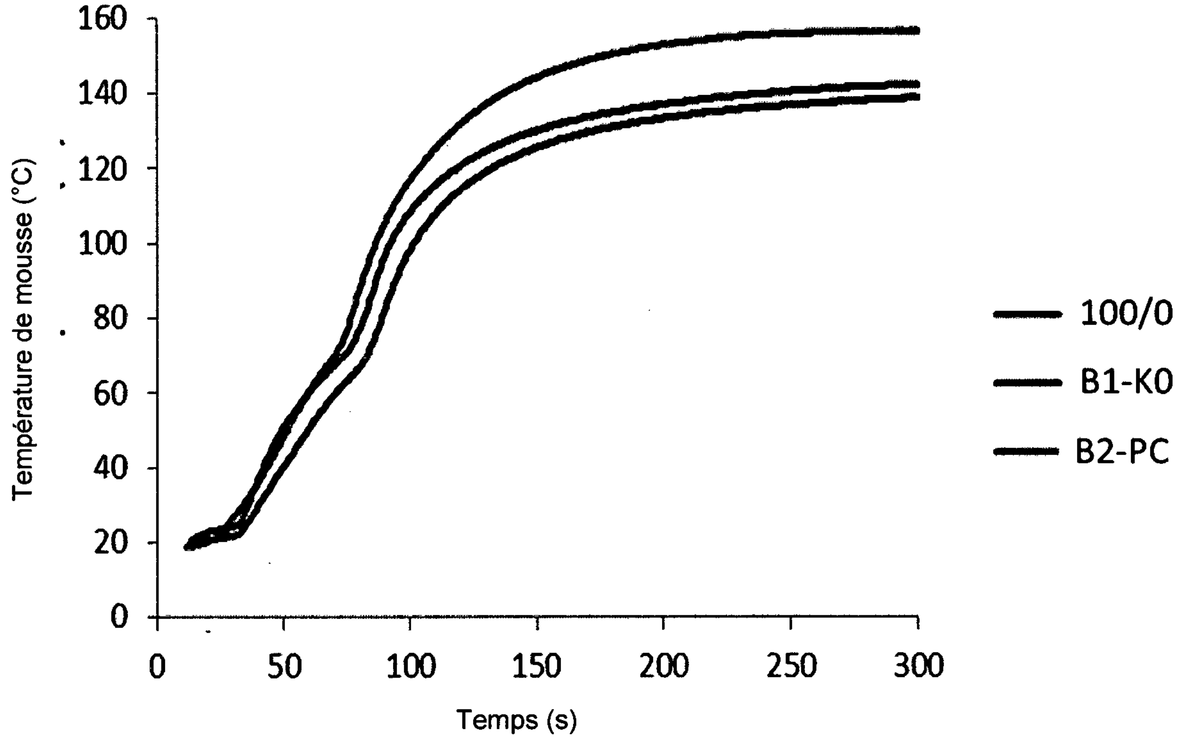
160
140
120
100
80
60
40
20
0
Température de mousse (°C)
0
50
100
150
200
250
300
Temps (s)
100/0
B1-K0
B2-PC

**Fig. 2**

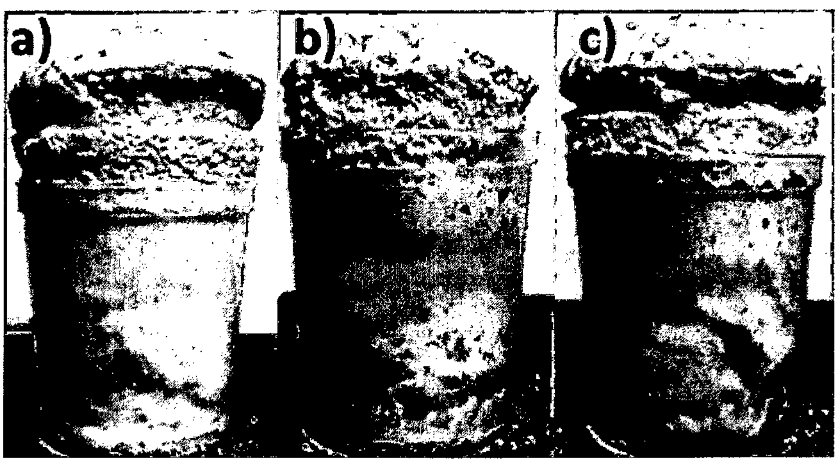
a)
b)
c)

**Fig. 3**

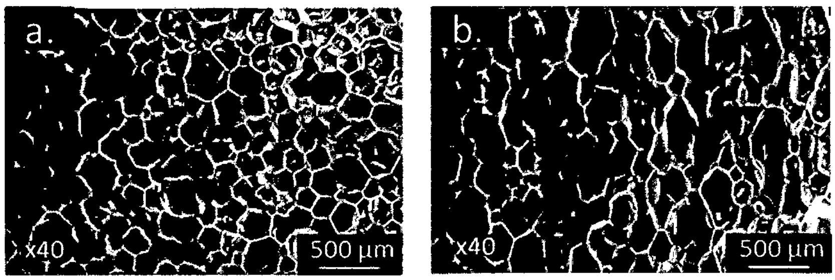
a.
x40
500 µm
b.
x40
500 µm

**Fig. 4**

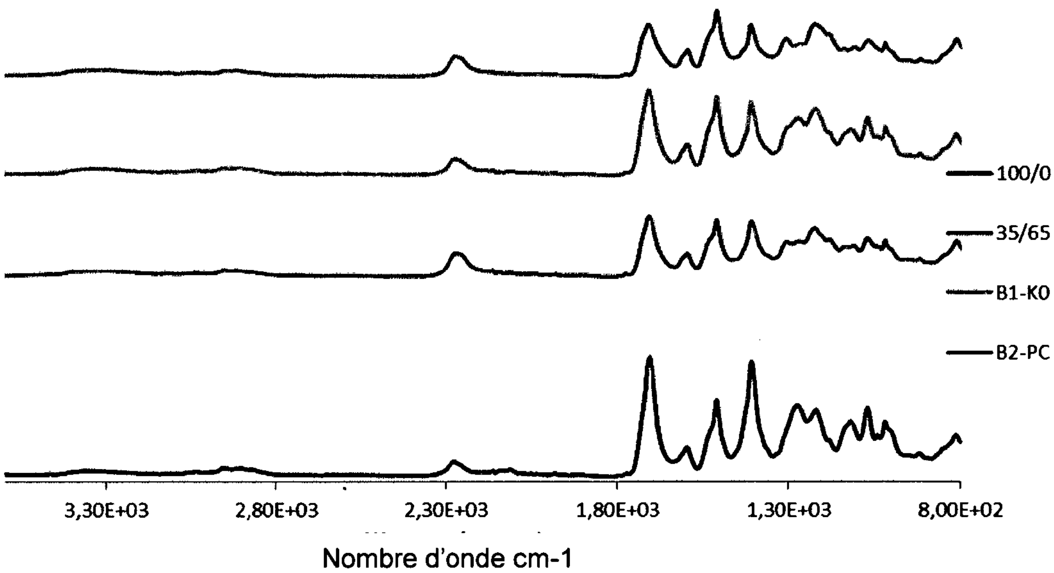
100/0
35/65
B1-KO
B2-PC
3,30E+03
2,80E+03
2,30E+03
1,80E+03
1,30E+03
8,00E+02
Nombre d'onde cm-1

**Fig. 5**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 4404295 A
- FR 1601253


### Littérature non-brevet citée dans la description


- **H. FLEURENT ; S. THIJS.** *J. Cell. Plast.,* 1995, vol. 31, 580-599
- **N. J. MILLS.** *J. Cell. Plast.,* 2011, vol. 47, 173-197
- **J. ANDERSONS et al.** *Mater. Des.,* 2016, vol. 92, 836-845
- **M. C. HAWKINS.** *J. Cell. Plast.,* 2005, vol. 41, 267-285
- **J. E. SHERIDAN ; C. A. HAINES.** *J. Cell. Plast.,* 1971, vol. 7, 135-139
- **H. E. REYMORE et al.** *J. Cell. Plast.,* 1975, vol. 11, 328-344
- **A. A. SEPTEVANI et al.** *Ind. Crops Prod.,* 2015, vol. 66, 16-26
- **I. JAVNI, Z. S. et al.** *J. Appl. Polym. Sci.,* 2000, vol. 77, 1723-1734